# EUROPEAN PATENT SPECIFICATION

(11) **EP 2 188 306 B1**
(45) Date of publication and mention of the grant of the patent: **29.06.2016**
(21) Application number: 08782610.3
(22) Date of filing: 04.08.2008
(51) Int. Cl.: C07K 16/06, C07K 16/42, G01N 33/68

(54) **IMMUNOGLOBULIN CLEAVAGE FRAGMENTS AS DISEASE INDICATORS AND COMPOSITIONS FOR DETECTING AND BINDING SUCH**
IMMUNOGLOBULINSPALTFRAGMENTE ALS KRANKHEITSINDIKATOREN UND ZUSAMMENSETZUNGEN ZUM NACHWEIS UND ZUR BINDUNG DAVON
FRAGMENTS DE CLIVAGE D'IMMUNOGLOBULINES UTILISÉS COMME INDICATEURS DE MALADIES, ET COMPOSITIONS PERMETTANT DE DÉTECTER ET DE FIXER CES FRAGMENTS

(30) Priority: 10.08.2007 US 955162 P
(43) Date of publication of application: 26.05.2010
(73) Proprietor: Janssen Biotech, Inc., Horsham, PA 19044 (US)
(72) Inventor: JORDAN, Robert, Radnor, PA 19087 (US); PETRONE, Diane, D., Radnor, PA 19087 (US); RYAN, Mary, Radnor, PA 19087 (US)
(74) Representative: Goodfellow, Hugh Robin
(86) International application number: PCT/US2008/072083
(87) International publication number: WO 2009/023457

(56) References cited:
- US-A1- 2005 119 464
- WELSCHOF ET AL: "The antigen-binding domain of a human IgG-anti-F(ab')2 autoantibody", PROCEEDINGS OF THE NATIONAL ACADEMY OF SCIENCES OF THE UNITED STATES (PNAS), NATIONAL ACADEMY OF SCIENCE, US, vol. 94, 1 March 1997 (1997-03-01), pages 1902-1907, XP002096050, ISSN: 0027-8424, DOI: DOI:10.1073/PNAS.94.5.1902
- TERNESS PETER ET AL: "The natural human IgG anti-F(ab')-2 antibody recognizes a conformational IgG1 hinge epitope", JOURNAL OF IMMUNOLOGY, vol. 154, no. 12, 1995, pages 6446-6452, XP002616031, ISSN: 0022-1767
- GEARING A J ET AL: "Selective cleavage of human IgG by the matrix metalloproteinases, matrilysin and stromelysin", IMMUNOLOGY LETTERS, ELSEVIER BV, NL, vol. 81, no. 1, 1 April 2002 (2002-04-01), pages 41-48, XP027288191, ISSN: 0165-2478 [retrieved on 2002-04-01]
- SCHMIDT A ET AL: "A synthetic peptide approach for elucidating the points of natural auto-antibody reactivity to proteolytic fragments of human IgG", BIOPOLYMERS, NEW YORK, NY, US, vol. 88, no. 4, Special issue, 1 January 2007 (2007-01-01), page 556, XP009142664, ISSN: 0006-3525
- ECKLE I ET AL: "Detection of granulocyte elastase specific IgG split products in rheumatoid synovial fluid", ADVANCES IN EXPERIMENTAL MEDICINE AND BIOLOGY, SPRINGER, US, vol. 240, 1 January 1988 (1988-01-01), pages 531-534, XP009142444, ISSN: 0065-2598
- GEARING ET AL: 'Selective cleavage of human lgG by the matrix metalloproteinases, matrilysin and stromelysin' IMMUNOLOGY LETTERS vol. 81, no. 1, April 2002, pages 41 - 48, XP027288191
- NASU H ET AL: 'Characterization of anti-F(ab')2 antibodies in SLE patients evidence for cross-reacting autoanti-idiotypic antibodies' CLINICAL IMMUNOLOGY AND IMMUNOPATHOLOGY vol. 25, no. 1, October 1982, pages 80 - 90, XP022958547
- WELSCHOF ET AL: 'The Antigen Binding Domain of Non-idiotypic Human Anti-F(ab')2 Autoantibodies: Study of their Interaction with lgG Hinge Region Epitopes' HUMAN IMMUNOLOGY vol. 60, no. 4, April 1999, pages 282 - 290, XP008131561
- WELSCHOF ET AL: 'The antigen-binding domain of a human lgG-anti-F(ab')2 autoantibody' PROC. NAT. ACAD. SCI. vol. 94, March 1997, USA, pages 1902 - 1907, XP002096050
- ECKLE ET AL: 'Detection of Granulocyte Elastase Specific lgG Split Products in Rheumatoid Synovial Fluid' ADV EXP MED BIOL vol. 240, 1988, pages 531 - 534, XP009142444
- YANO ET AL: 'Natural antibodies against the Immunoglobulin F(ab')2 fragment cause elimination of antigens recognized by the F(ab')2 from the circulation' EUR J IMMUNOL vol. 25, no. 11, November 1995, pages 3128 - 3133, XP008131562
- RYAN ET AL: "Proteolysis of purified IgGs by human and bacterial enzymes in vitro and the detection of specific proteolytic fragments of endogenous IgG in rheumatoid synovial fluid", MOLECULAR IMMUNOLOGY, PERGAMON, GB, vol. 45, no. 7, 21 December 2007 (2007-12-21), pages 1837-1846, XP022478946, ISSN: 0161-5890, DOI: 10.1016/J.MOLIMM.2007.10.043

## Description

### BACKGROUND OF THE INVENTION

### Field of the Invention

The invention relates to diagnostic and prognostic indicators and methods and reagents for their detection. The invention further relates to methods of monitoring the natural history of disease in a patient.

### Description of the Related Art

In medicine, a biomarker is a biochemical substance that can be used to measure the progress of disease or the effects of treatment, that is, a diagnostic or prognostic indicator. One biomarker which effectively reflects the natural history of disease and disease control is hemoglobin A1c for glycemic control in diabetic patients. Due to the long half-life of HbA1c in serum, it serves as a recent record of the excursion of blood glucose away from ideal levels as well as the duration of such excursions. A currently used biomarker of systemic inflammatory conditions is C-reactive protein (CRP) (Pepys MB et al. J. Clin. Invest. 111; 1805-1812, 2003). CRP is an acute phase reactant that is produced in response to a wide variety of acute inflammatory conditions. CRP is synthesized in the liver in response to cytokine signals including TNF and IL-6 which themselves migrate from the distant site of inflammation. An increase in serum of CRP occurs in infection, stroke, vascular disease, myocardial infarction and several other acute inflammatory disorders.

Circulating immunoglobulins, and specifically those antibodies of the IgG class, are major serum proteins. It is well-known that human proteases are associated with inflammatory, proliferative, metastatic, and infectious diseases. Human proteases such as matrix metalloproteinases (MMPs) and neutrophil elastase cleave the IgGs heavy chain polypeptide at a residue unique to each protesase as do bacterial proteases such as glutamyl endopeptidase (*Staph. aureus*) or immunoglobulin degrading enzyme of streptococcus (*Strep. pyogenes*). The cleavage sites in the heavy chain are clustered around the region termed the hinge domain, where the interchain disulfide linkage of the two heavy chains occurs. The region below the hinge constitutes the Fc region and comprises binding sites responsible for the effector functions of IgG. In the case of microorganisms, protease expression is a potential adjunctive virulence pathway allowing organisms to avoid opsonization (Rooijakkers et al. Microbes and Infection 7: 476-484, 2005) in so far as the proteolytic release of the Fc domain by cleavage below the hinge effectively neutralizes functions that would otherwise lead to the targeting and killing of that pathological cell. Thus, the elaboration of specific proteases may be representative of a myriad of diseases states including cancer, inflammation and infectious diseases.

That IgG degradation is enhanced in pathologic *in vivo* environments as evidenced by the presence of natural IgG autoantibodies that bind to the cleaved hinge domain (Knight et al., 1995; Nasu et al., 1980; Persselin and Stevens, 1985, Terness, et al. 1995 J Imunol. 154: 6446-6452). These autoantibodies also bind the Fab and F(ab')₂ fragments generated by several proteinases (including papain and pepsin), with particularly strong reactivity to the lower hinge domain remaining as C-terminal residues in F(ab')2 molecules (Terness et al., 1995). The detection of the actual cleavage products have been reported (Fick et al., 1985; Goldberg and Whitehouse, 1970; Waller, 1974) but a robust assay which would allow these fragments to serve as biomarkers has not been developed possibly due to the low concentrations in serum resulting from rapid clearance of the various fragments or to technical problems in detecting the fragments amidst the large amount of intact immunoglobulin in blood and tissues. A specific antibody was prepared (Eckle, et al. 1988. Adv. Exp. Med. Biol. 240: 531-534) for detection of human neutrophil elastase cleaved Fc domain which detected Fc at a median concentration of 0.62 ug/ml directly in synovial fluid of rheumatoid arthritis patients but not in synovial fluid from patients with other types of joint disease.

Welschof et al. (1997) PNAS 94:1902-1907 describes an antigen-binding domain of a human IgG-anti(Fab')2 autoantibody.

Gearing et al. (2002) Immunology Letters 81(1):41-48 describes selective cleavage of human IgG by matrix metalloproteinases.

Schmidt et al. (2007) Biopolymers 88(4):556 describes a synthetic peptide approach for elucidating the points of natural auto-antibody reactivity to proteolytic fragments of human IgG.

Ryan et al. (2008) Molecular Immunology 45:1837-1846 describes proteolysis of purified IgGs by human and bacterial enzymes *in vitro*.

Therefore, the ability to assess the type and amount of IgG cleavage product(s) in the bodily fluids or blood of subjects could be used as a biomarker of specific disease activity. Specific reagents and methods for such determinations would provide useful tools for diagnostic and prognostic medical assays.

### SUMMARY OF THE INVENTION

The invention provides an antibody composition that comprises at least one antibody that specifically binds to an IgG protease cleavage product characterized by, a) having molecular weight which is comparable to an intact mammalian IgG under non-denaturing conditions and b) being separable into two fragments which comprise an antigen binding fragment of 135 kDa and a CH2-containing fragment under denaturing but non-reducing conditions and c) wherein the reagent does not react with intact IgG,
and wherein said antibody specifically binds to a protease specific cleavage site in human IgG1 produced by IdeS, TCPPCPAPELLG.

The invention further provides a human IgG protease cleavage site peptide analog consisting of TCPPCPAPELLG covalently attached to keyhole limpet hemocyanin via the N-terminus.

The invention further provides a method of preparing of the antibody of the invention using a non-human animal immunized with the peptide analog of the invention, wherein the reagent is purified from the serum of said animal by pre-absorption on a human IgG affinity matrix.

The invention further provides a method of detecting a disease process in a subject by analysis of a tissue sample from the subject, wherein said method comprises the detection by the antibody composition of the invention of an IgG proteolytically cleaved by IdeS characterized by a) having a molecular weight which is comparable to an intact mammalian IgG under physiological conditions and b) being separable into two fragments which comprise an antigen binding fragment of 135 kDa and a CH2-containing fragment under denaturing but non-reducing conditions.

The invention further provides a kit including a reagent for detection of a disease marker in tissue of a subject, which reagent comprises at least one antibody that specifically binds to a cleaved IgG, which antibody is capable of detecting an IgG cleavage product characterized by a) having molecular weight which is comparable to an intact mammalian IgG under non-denaturing conditions and b) being separable into two fragments which comprise an antigen binding fragment of 135 kDa and a CH2-containing fragment under denaturing but non-reducing conditions and c) wherein the reagent does not react with intact IgG,
and wherein said antibody specifically binds to a protease specific cleavage site in human IgG1 produced by IdeS, TCPPCPAPELLG.

The invention further provides the antibody composition of the invention for use in therapy.

The invention relates to reagents and use of the reagents to detect a disease process associated with elaboration of proteases, which proteases are manifestations of the disease pathology as well as agents which limit host immunological defenses. In one aspect of the invention, the reagents and use of the reagents in an assay, detects anti-disease antibodies, which antibodies are specific for targets related to the disease pathology. The reagents are directed to assessing an IgG breakdown product that is the result of such proteolytic cleavage.

In another embodiment, the methods of the disclosure are directed to detection of an IgG cleavage product which is characterized by 1) having a molecular weight which is comparable to an intact mammalian IgG under physiological conditions and 2) being separable into two fragments which comprise an antigen binding fragment and a 32 kDa fragment under denaturing but non-reducing conditions and 3) does not exhibit ADCC activity in an in vitro assay. In one aspect of the method of detecting the IgG cleavage product of the invention, a specific reagent capable of detecting the cleavage product is provided, which reagent is at least one antibody capable of binding to said cleavage product.

In another embodiment of the disclosure, the sequences for generating the reagents useful for detection of the IgG cleavage product are provided which are useful for immunizing, panning, and selection of the anti-IgG cleavage product reagent of the disclosure. In one aspect, the sequence is selected from the group consisting of at least 5 contiguous amino acids selected from the human IgG hinge region sequences of SEQ ID NO: 1, 2, 3, or 4 that are on the amino terminal side of a protease cleavage site. In one embodiment, the sequences are selected from those of SEQ ID NOs. 5-11 and N terminal truncations thereof. In another aspect, a method of designing a peptide immunogen based on the proteolytic cleavage site of a human IgG molecule is provided.

Herein disclosed are methods of preparation of an anti-IgG cleavage product antibody of the disclosure including nucleic acid sequences, vectors, and host cells for the recombinant production of anti-IgG cleavage product antibodies. In another aspect of the method of manufacturing the anti-IgG cleavage product antibodies, immune host animals are disclosed which animals' serum is a source of the antibodies of the disclosure from which the reagent is prepared by methods described or known in the art.

In another embodiment of the invention, a kit for detection of anti-IgG cleavage product is provided comprising anti-IgG cleavage product antibodies of the invention.

Herein also disclosed is a method of administering an anti-IgG cleavage specific antibody to treat a patient in order to restore effector functions to an IgG cleavage product.

### BRIEF DESCRIPTION OF THE SEVERAL VIEWS OF THE DRAWING

**FIG. 1** depicts the various domains of a typical mammalian IgG class antibody showing their relationship to the hinge and the pepsin and papain cleavage products defined as Fab, F(ab')₂, and Fc.
**Fig. 2** shows four individual Agilent Biosizing microcapillary electrophoresis analyses as gel images at times during each of the proteinase digests of a human IgG1κ antibody, Mab1, by 1% w/w of human MMP-3 (A), streptococcal IdeS (B), staphylococcal glutamyl endopeptidase I (C) and human neutrophil elastase (D) at 37 °C. Standards on each gel (lane 1) correspond to an intact human/murine chimeric IgG1 and known cleavage fragments.
**Fig. 3** shows the sequence of the human IgG1 heavy chain around the hinge region; the positions of major proteolytic cleavages are indicated by arrows.
**Fig. 4** is a western blot showing the time course of biotinylated murine/human IgG degradation by wound exudate.

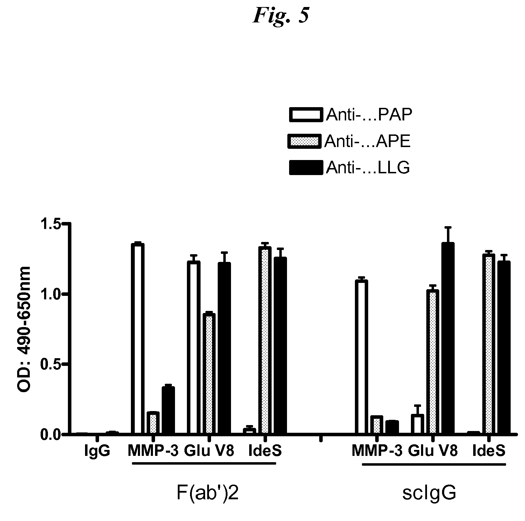

**Fig. 5** a graph showing the relative specificity of the antisera generated in rabbits immunized with conjugated F(ab')₂ degradation product from three different proteases: MMP-3, V8, and IdeS TCPPCPAP, residues 7-14 of SEQ ID NO: 1 corresponding to the MMP-3 cleavage site, TCPPCPAPE, residues 7-15 of SEQ ID NO: 1 corresponding to the glutamyl endopeptidase site, and TCPPCPAPELLG, residues 7-18 of SEQ ID NO: 1 corresponding to the IdeS site. ELISA reactivity of three individual rabbit polyclonal anti-hinge peptide antibody preparations with F(ab')₂ fragments of Mab3 IgG1κ. The F(ab')₂ fragments were generated with human recombinant MMP-3, staphylococcal glutamyl endopeptidase I and recombinant IdeS from *Strep. pyogenes.* A mixed pool of the rabbit antibody preparations demonstrated nearly equivalent reactivity with each of the Mab3 F(ab')₂ fragments. Bars correspond to the mean ± standard deviation of three replicate wells.
**Fig. 6** Western blot reactivity of rabbit polyclonal antibody preparations with antibody digests. Mab3 human IgG1 intact or that had been partially digested with MMP-3, glutamyl endopeptidase (V8) or IdeS was separated by SDS-PAGE followed by immunoblotting. (A) Blotted with anti-human IgG (H+L) [lanes 1-4] or Anti...LLG rabbit polyclonal [lanes 6-10]. (B) Blotted with Anti...PAP [lanes 2-5] or Anti...APE [lanes7-10]. Blots were cut prior to incubation with antibodies through lane 5 in panel A and lane 6 in panel B to allow for detection with the individual antisera.
**Fig. 7** is a western blot developed using the RAH-1 reagent on samples from an analysis of IgG degradation in the synovial fluid from 5 RA patients and compared to samples from *in vitro* proteolytic digests of a monoclonal IgG1 with MMP-3, glutamyl endopeptidase (V8) and IdeS.
**Fig. 8** shows the serum concentration over time of various proteolytic cleavage fragments, intact IgG, scIgG and F(ab')₂; after injection of the specified purified fragments into mice, measured using the goat anti-human IgG (H+L).
**Fig. 9** is a dot plot of the individual values of scIgG detected by the reagent RAH-1 in human serum samples from patients diagnosed with the diseases as indicated as compared to that in a group of normal human serum samples where the lines indicated mean values in each group.
**Fig. 10** shows the concentration of scIgG in diluted serum of 10 individuals with rheumatoid arthritis (RA) and an equal number of healthy, normal controls detected with reagent RAH-1 using an improved version of the ELISA; "(2)" in the RA group signifies that the same value was obtained in two separate individuals.
**Fig. 11** shows the relative reactivity of rabbit monoclonal antibody targeting cleavage fragments from human IgG1 hinge as peptide analogs and to antibody fragments terminating at the residue specified (see Fig. 3).
**Fig. 12** shows the concentration dependence of three different rabbit monoclonal antibodies targeting cleavage fragments of human IgG1 hinge in restoring complement-dependent cell lysis (CDC) to F(ab')₂ created by digesting IgG1 with IdeS compared to a rabbit polyclonal prepared to cleavage peptide analogs (rb poly).

### BRIEF DESCRIPTION OF THE SEQUENCE LISTING

| SEQ ID NO: | DESCRIPTION |
|---|---|
| 1 | Human IgG1 hinge region |
| 2 | Human IgG4 hinge region |
| 3 | Human IgG2 hinge region |
| 4 | Human IgG3 hinge region |
| 5 | MMP-3 and MMP12 cleavage peptide |
| 6 | Glutamyl endopeptidase 1 and Cathepsin G cleavage peptide |
| 7 | IdeS cleavage peptide |
| 8 | Plasmin cleavage peptide |
| 9 | HNE cleavage peptide |
| 10 | Pepsin and MMP-7 cleavage peptide |
| 11 | Papain cleavage peptide |

### DETAILED DESCRIPTION OF THE INVENTION

### Abbreviations

Abs = antibodies, ADCC =antibody-dependent cell-mediated cytotoxicity; CDC =complement directed cytoxicity; ; HNE =human neutrophil elastase; IdeS = immunoglobulin degrading enzyme of *S. pyrogenes;* Ig = immunoglobulin; Mab = monoclonal antibody; MMP = matrix metalloprotease; scIgG = single cleaved IgG; SA = streptavidin; V8 = glutamyl endopeptidase I from *Staph. aureus.*

### Definitions

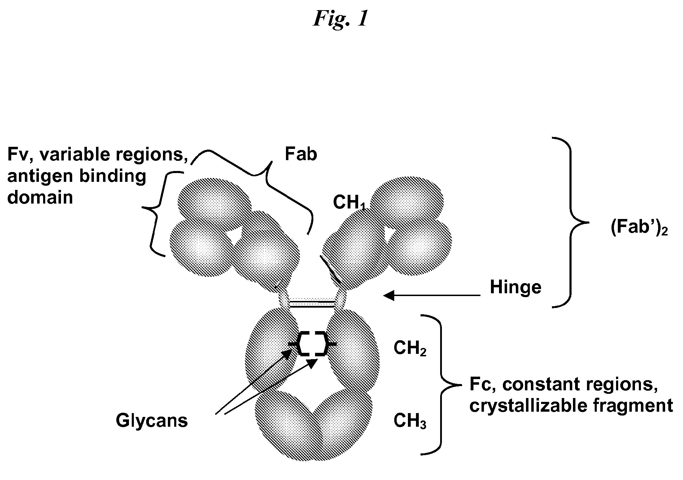

Antibody fragments; Fab, F(ab')₂, and Fc are terms describing proteolytic cleavage products of IgG antibodies which may be further dissociated by reduction of the disulfide bonds between the heavy chains (the core hinge region). Classic proteolytically generated antibody fragments, include: Fab (e.g., by papain digestion), Fab' (e.g., by pepsin digestion and partial reduction) and F(ab')₂ (e.g., by pepsin digestion), facb (e.g., by plasmin digestion), pFc' (e.g., by pepsin or plasmin digestion), Fd (e.g., by pepsin digestion, partial reduction and reaggregation), where reduction removes the disulfide linkage between cysteine residues forming interchain linkages (refer to Fig. 1). As the Fc fragment was described as a papain cleavage fragment and which cleaves human IgG1 at residue 224 (EU numbering), which is N-terminal to the hinge, the Fc fragment is assumed to retain the hinge and the disulfide linkages between the heavy chains, however, due to the high degree of association between the heavy chain CH2-CH3 dimers in the antibody, a dimeric structure is retained even in the absence of the disulfide (hinge) bonds. Thus, as used herein "Fc" refers to the dimeric structure formed by association of the heavy chain CH2-CH3 segments whether covalently bound or not. It will be understood that the non-covalently associated Fc may be distinguished from the disulfide linked Fc by its ability to undergo dissociation into CH2-CH3 monomers in the presence of a denaturant such as a detergent.

The terms "proteolytic", "proteolytic cleavage", "protease", and "proteolytic enzyme" are used interchangeably and mean an agent, e.g. enzyme, which is able to cleave a polypeptide chain producing two or more fragments, where the enzyme acts under normal temperature and under physiological conditions or physiologically compatible conditions. Physiological conditions include any temperature, buffer, cation, anion, substrate, catalyst, pH, cofactor, or the like which is a naturally found in the body of a living mammal whether in health or disease. However, the protease may be derived from a non-mammalian source such as from a pathogen which may be of any type of life form.

By "scIgG" or "single cleaved IgG" is meant any immunoglobulin class G molecules having a heterodimeric structure comprising two heavy chains and two light chains, where one of the heavy chains has been subjected to proteolytic cleavage on a single heavy chain while the second heavy chain remains intact.

By "upstream" relative to an amino acid sequence written from the N-terminal to the C-terminal residue is meant the residues in the sequence towards the N-terminus from a given residue. Conversely, by "downstream" relative to an amino acid sequence is meant the residues in the sequence towards the C-terminus from a given residue.

### Antibody Functions by Substructure

In general, immunoglobulins, antibodies, consist of regions of continuous polypeptide chain comprising approximately 100 amino acids which show a characteristically folded globular domain and represent different elements of the structure. Each 100 of the amino acid regions represents a globular domain which is about 10-11 kDa. For immunogammaglobulins (IgGs), these domains are grouped together into segments; the Fab segment is comprised of a light chain variable joined to a light chain constant region in a single chain linked through a disulfide bond to the heavy chain first constant region (CH1) which is contiguous with the heavy chain variable region; Fc is comprised of two contiguous heavy chain constant regions (CH2 and CH3) linked through two or three disulfide bonds in the hinge region. Studies have shown that proteases, such as papain and pepsin, preferentially cleave antibodies at sites which are between the segments. Two identical Fab segments connected via the hinge region to one Fc segment, thus form a Y-shaped conformation of the 150 kDa structure (see Fig. 1). Fab segments generated using papain typically have a molecular weight of 46 kDa, nonreduced F(ab')₂ typically have a molecular weight of 90-100 kDa, and nonglycosylated, nonreduced Fc will have an apparent molecular weight of approximately 50-60 kDa. However, as each antibody species, and each subclass of antibody within a species, is slightly different, the exact nature and location of the cleavage and cleavage products are variant.

Antigen binds to antibodies via an antigen binding site in the variable domains of each pair of light and heavy chains (Fig. 1). Other molecules, known as effector molecules or cells, bind to other sites in the remainder of the molecule, ie other than the antigen binding sites, and this portion of antibody includes the more invariant immunoglobulin sequences, "the constant portion" of an antibody, such sites being located particularly in the Fc region constituted by the portions of the heavy chains extending beyond the ends of the light chains.

Antibodies have several effector functions mediated by binding of effector molecules. For example, binding of the C1 component of complement to antibodies activates the complement system. Activation of complement is important in the opsonisation and lysis of cell pathogens (a process called complement-mediated cytotoxicity or CDC). The activation of complement stimulates the inflammatory response and may also be involved in autoimmune hypersensitivity. Further, antibodies bind to cells via the Fc region, with a Fc receptor site on the antibody Fc region binding to a Fc receptor (FcR) on a cell. There are a number of Fc receptors which are specific for different classes of antibody, including IgG (gamma receptors), IgE (eta receptors), IgA (alpha receptors) and IgM (mu receptors). Binding of antibody to Fc receptors on cell surfaces triggers a number of important and diverse biological responses including engulfment and destruction of antibody- coated particles, clearance of immune complexes, lysis of antibody-coated target cells by killer cells (called antibody-dependent cell-mediated cytotoxicity, or ADCC), release of inflammatory mediators, placental transfer and control of immunoglobulin production.

The sequences around the hinge domain are conserved among IgG isotypes (SEQ ID NO: 1-4) and among mammalian species generally. The IgG1 (SEQ ID NO: 1) and IgG3 (SEQ ID NO: 1) isotype comprise a Leu-Leu pair that is a structural motif for binding to Fcγ receptor(s) and for Fc effector functions. Other residues downstream of the "hinge core" which typically comprises at least cysteine separated by two non-cysteine residues, are also conserved.

Applicants have discovered that a cleavage product, scIgG, of human IgG1 is formed by human and bacterial proteases when proteolysis occurs on one of the two heavy chain polypeptides that comprise an IgG, while not disrupting the overall composition of the heterodimeric molecule. Secondly, applicants have determined through kinetic analysis of proteolytic attack on human heavy chain constant region-containing IgG molecules, that scIgG is likely the more abundant product of *in* vivo proteolysis. The existence of scIgG as a proteolytic intermediate leading to F(ab')₂ during proteolysis of IgG has been noted previously for the MMP-3 enzyme (Gearing AJH et al, Immunol. Lett. 81: 41-48, 2002). Cleavage of IgG by a streptococcal protease, IdeS, was also noted to produce a product resembling intact IgG by size-exclusion chromatography (Vincents B et al, Biochemistry 43: 15540-15549, 2004). However, no functional characterizations of this intermediate were reported nor were methods for detecting the scIgG in biological samples provided.

Applicants have further demonstrated that, in vivo, scIgG, exhibits a serum half-life compatible with assessment of disease activity over a period of several days to months, thereby enabling the use of scIgG as a marker of latent or suppressed disease processes, or could be used to understand the recent natural history and response or recovery from a disease.

Briefly, applicants have discovered that the kinetics of proteolytic cleavage under physiological conditions lead to a larger proportion of proteolytically cleaved IgG being in the scIgG conformation than species which are products of multiply cleavage events, such as the F(ab')₂ (see Fig. 1). In the process of testing a large number of proteases, it was determined that the first cleavage of a heavy chain constant region in an intact IgG proceeds more rapidly than the second, a sequence that leads to a temporal accumulation of the singly cleaved species. This single cleaved version of the IgG molecule is indistinguishable from its intact parent in many ways (e.g. molecular size, antigen binding, ability to be recognized by protein A/G).

In accordance with the invention applicants have generated reagents suitable for the detection of proteolytic cleavage products including F(ab')₂ and scIgG. The reagents of the invention generated using cleavage site analog peptides of the invention, recognize human IgG1 cleavage products but do not recognize intact IgG.

Applicants have further demonstrated that antibodies recognizing IgG cleavage products retaining antigen binding specificity can restore effector functions such as CDC and ADCC to the cleaved IgG..

### Proteolytic Enzymes and Disease Association

Applicants demonstrate that antibody cleavage products, including scIgGs, similar in size to those generated with *in* vitro enzyme panel, are detectable in inflammatory exudates such as synovial fluid from patients with rheumatoid arthritis. Further, scIgG can be detected in the serum of patients with a number of diseases in which localized proteolytic activity is a known characteristic of the pathology. The scIgG in these disease states is at higher concentrations than in healthy normal volunteers and is also higher than in the serum of patients with less severely inflammatory disease.

The detection of scIgG was enabled by the generation of affinity-purified polyclonal antibodies (rabbit) that specifically bind to newly exposed epitopes in the cleaved heavy chain at or around the hinge disulfides, but do not react with the intact, non-cleaved IgG molecule. Confirmatory support for the detection of scIgG in serum is its prolonged circulating lifespan similar to intact IgG. The ability to detect scIgG in the bodily fluids or blood of diseased individuals is a potentially novel biomarker strategy. It will be understood that other species of antibody besides rabbit (such as mouse, rat, and camel) may be used and monoclonal antibodies produced, for example, by cloning of a antibody gene coding for a specified antibody binding region sequence which polyclonal or monoclonal antibody retains the ability to bind human IgG1 cleavage products but that do not recognize intact IgG are encompassed as reagents of the invention. Other methods of producing antibodies, e.g. by selection from antibody domain libraries, are well known to those skilled in the art and may be used as a source of the antibodies of the invention.

### Antibody Reagents

The antibodies of this invention can be prepared in several ways well known in the art using criteria and immunogens designed by applicants to raise or select antibodies useful in the practice of the invention.

In one aspect, the antibodies are conveniently obtained from hybridomas prepared by immunizing an animal with the observed cleavage fragments or cleavage site analog peptides derived therefrom. Thus, the antibodies can be obtained by immunizing animals or screening antibody libraries with antibody cleavage fragments including F(ab')₂ and scIgG, or N-terminal truncations or structural analogs thereof. In one embodiment, the peptides used for generating the antibodies are selected from the 14-mer peptides fragments of IgG1 shown in SEQ ID NO: 5-11, where the C-terminal residue of the polypeptide or peptide represents the residue upstream (N-terminal side) of the cleavage site as shown in Table 1 of the residue cleavage pairs. Fragments comprising the hinge motif, e.g. -T-C-P-P-C- of IgG1 (residues 7-11 of SEQ ID NO: 1), will be multimeric due to disulfide bond formation, unless the cysteine residues (C) have been replaced with e.g. alanine (A) residues.

In a specific embodiment, the antibody is generated using an 8-mer peptide corresponding to the sequence of amino acids on the amino terminal side of the MMP-3 cleavage site (TCPPCPAP, residues 7-14 of SEQ ID NO: 1), or extended peptides corresponding to the glutamyl endopeptidase site (TCPPCPAPE, residues 7-15 of SEQ ID NO: 1); or the IdeS site (TCPPCPAPELLG, residues 7-18 of SEQ ID NO: 1). When used as immunogens, the peptides can conveniently be covalently attached to keyhole limpet hemocyanin (KLH) via the N-terminus or through an added linker residue or peptide.

The antibodies can thus be obtained using any of the hybridoma techniques well known in the art, see, e.g., Ausubel, et al., ed., Current Protocols in Molecular Biology, John Wiley & Sons, Inc., NY, NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY, NY, (1997-2001). An antibody of the invention can include or be derived from any mammal, such as but not limited to a human, a mouse, a rabbit, a rat, a rodent, a primate, or any combination thereof and includes isolated human, primate, rodent, mammalian, chimeric, humanized and/or CDR-grafted anti-integrin antibodies, immunoglobulins, cleavage products and other specified portions and variants thereof.

Phage-displayed antibody libraries may also be used to identify novel binding domains with the desired specificity to scIgG and other antibody fragments.

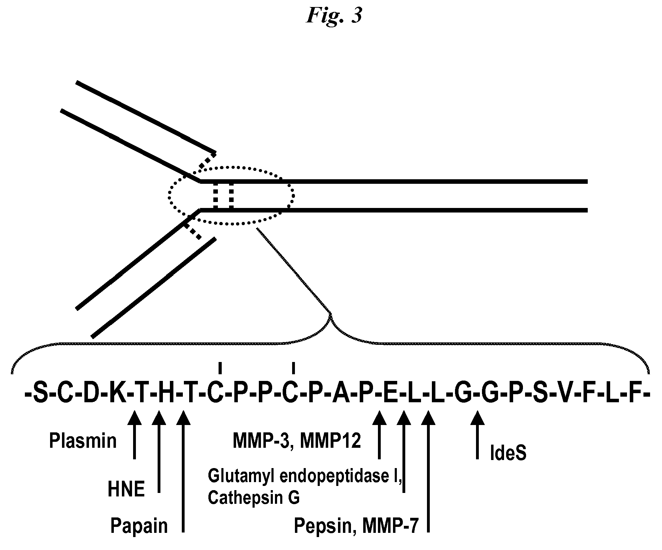

In raising or selecting antibodies or other binders useful in the present invention, the specific reagents used for this purpose are a further aspect of the invention. The specific immunogens or test reagents developed for this purpose are characterized as comprising residues around the hinge core of the IgG1, including but not limited to the residues SCDKTHTCPP CPAPELLGGP SVFLFP (SEQ ID NO: 1) as shown in Fig 3. Hinge regions of other human isotype antibodies that produce antibody fragments upon contact with proteolytic enzymes may also serve as sources of analog for the purposes of creating, selecting or testing antibodies or other binding molecules to enzymatic cleavage products. An analogous region of the human IgG4 heavy chain includes residues TCNVDHKPSN TKVDKRVESK YGPPCPSCPA PEFLGGPSVF LF (SEQ ID NO: 2) and for IgG2 and IgG3 as shown in SEQ ID NOS: 3 and 4, respectively. In each case, the peptides consist of at least 5 contiguous amino acids selected from the human IgG hinge region sequences of SEQ ID NO: 1, 2, 3, or 4 that are on the amino terminal side of a protease cleavage site. In one aspect, the specific immunogen or peptide used for generating the antibodies comprise at least the hinge core of the IgG1, defined as the residues -T-C-P-P-C-. In a specific embodiment, the peptide is a 12-mer peptide analog of the human IgG1 lower hinge and adjoining CH2 domain having the sequence TCPPCPAPELLG (residues 7-18 of SEQ ID NO: 1). A general method for creating peptide fragments useful in creating, selecting or testing antibodies or other binding molecules to enzymatic cleavage products is to a) identify the N-terminal residue of a pair of residues of an antibody heavy chain cleaved by a protease as exemplified by specific proteases in Example 1 and shown in Table 1, b) define from 5-14 or more upstream residues from that cleavage site where the N-terminal residue will become the C-terminus of the defined sequence and c) produce the peptide in sufficient amounts for the desired purpose(s). Peptides such as those described are those selected from SEQ ID NO: 5-11 or N-terminal truncations thereof. The peptides may be labeled, conjugated or cross-linked or used in admixture one with another or with adjuvants for the purposes of testing binding or as immunogens or panning targets for use e.g. in selecting binders from a phage display library.

Herein disclosed, in one aspect, are isolated nucleic acid molecules comprising, complementary, or hybridizing to, a polynucleotide encoding the aforementioned specific peptides or antibodies thereto, comprising at least one specified sequence, domain, portion or variant thereof. The present disclosure encompasses isolated nucleic acids encoding at least one isolated monoclonal antibody having specificity for the scIgG as described herein and a nucleic acid vector comprising the isolated nucleic acid, and/or a prokaryotic or eukaryotic host cell comprising the isolated nucleic acid. The host cell can optionally be at least one selected from E. Coli, COS-1, COS-7, HEK293, BHK21, CHO, BSC-1, Hep G2, 653, SP2/0, 293, HeLa, myeloma, lymphoma, yeast, insect or plant cells, or any derivative, immortalized or transformed cell thereof. Also disclosed is a method for producing at least one antibody of the disclosure, comprising translating the antibody encoding nucleic acid under conditions in vitro, in vivo or in situ, such that the peptide or antibody is expressed in detectable or recoverable amounts.

### Methods of Use

The reagents of the invention are useful in detecting disease pathology when the disease process invokes, is a result of, causes, or is otherwise associated with proteolytic activity and proteolytic enzymes, proteases. Such diseases and processes include those precipitating or aggravating, produced by, or resulting from infection, stroke, vascular disease, myocardial infarction and several other acute and chronic inflammatory disorders. Applicants have demonstrated that one particularly useful biomarker of the proteolytic activity is scIgG, which is detected at increased levels in some of the aforementioned disorders. As scIgG is generated locally at the site of the pathology or pathological process or infection, scIgG provides a unique and specific marker of such processes as a gauge of the involvement of specific tissues or cell types at the disease site.

In one embodiment of the method of the invention, a sample is obtained from a subject suspected of having, having had, or having been treated for a disease characterized by elevated levels of proteases. The sample is contacted with a binding agent, such as an antibody preparation, having specificity for IgG cleavage fragments known to result from contact between the disease stimulated protease and a population of serum IgG.

The method of the invention can be used to assess whether patients previously diagnosed with a disease or condition are at risk for advanced disease (e.g. cancer metastases, aggressive tumor growth, persistent infection, etc.).

In some cases, for example in the cancer patient, the detection of scIgG may be useful to indicate advanced disease progression involving metastatic spread which is known to involve elaboration of proteolytic enzymes, especially MMPs. In some aspects, neoplastic disease shares these mechanisms generally with inflammatory processes, tissue repair, and healing (Coussens, L.M. and Werb, Z. 2002. Nature 420 (19): 860-867). Other studies have shown that, for example, lipid lowering correlates both with a reduction in the risk for cardiac and vascular events, e.g. thrombosis, and with a reduction in MMPs such as MMP-2 and MMP-9 and that these enzymes are produced by atherosclerotic plaques (Deguchi, J, Maanori, A., Ching-Hsuan, T. et al. 2006 Circulation 114: 555-62). Thus, the methods of the invention are particularly applicable but not limited to patients with severe arthritic syndromes (RA, ankylosing spondylitis), certain cancers (especially inflammatory breast cancer), severe coronary arterial settings (myocardial infarction and congestive heart failure) and other diseases like asthma. The method of the invention may be used to distinguish those diseases and conditions in which the pathophysiology involves or induces protease capable of acting upon IgG from other pathologies not characterized by enhanced elevated levels of secreted proteases or wherein the proteases do not cleave IgG.

Thus, while the method of using the reagents described herein are specific for detection of the cleaved fragments, more specific analysis of the cleaved fragments could include an analysis of the binding specificity of the variable regions of the cleaved antibody. For example, a solid phase assay which combines antigen binding selectivity with fragmented antibody detection could be used to determine whether certain antigens and proteases are co-localized in a subject thereby providing information about the nature of the tissue, disease, or pathology at the site of proteolytic activity.

Drawing blood is the most frequently practiced form of tissue sampling from subjects, human or animal, healthy or ill. In so far as scIgG is found systemically, and is not restricted to the site of formation, that is, the site of the protease activity, it is a reporter marker for disease activity which may localized in specific compartments. One such compartment is the synovial fluid. Thus, blood or serum collection provides a convenient and feasible source for detection of early disease using the reagents and methods provided by the present invention. Alternatively, sampling of local settings like RA synovial fluid, lung exudates, biopsies, and the like could also be applied to patients at any stage including diagnosis or in patients with advanced disease. Cleaved antibody fragments may be detected in such tissue samples by direct staining (immunohistochemical methods) or in fractioned samples derived from the samples.

Tissue samples, including blood, should be treated so as to inhibit any residual active proteases. Chelation of metals (e.g. EDTA) effectively inhibits MMPs. Iodoacetamide blocks cysteine proteases (e.g. IdeS), serine proteases can be blocked with DFP and similar compounds. Active proteases are present in synovial fluid and should be processed accordingly. Samples may also be maintained frozen until the time of assay. Once the samples have been appropriately processed, the scIgG specific reagents of the invention may be used in any antibody-based techniques such as ELISA, bead-based formats, RIAs, known to those skilled in the art or yet to be developed.

The anti-IgG proteolytic cleavage fragment reagents of the invention may be packaged in a kit for research or diagnostic use and for commercial sale along with other reagents such as buffers and standards such as intact human IgG and known quantitities of cleaved IgG along with instructions for the measurement and, if desired, quantitation of IgG proteolytic cleavage fragments in tissue samples harvest from subjects.

Antibodies of the invention immunospecific for hinge peptide cleavage fragments are capable of binding the remnants of enzymatically cleaved IgG which retain antigen binding domains, e.g. Fab, F(ab')₂, scIgG , and thus restore the Fc-related binding characteristics and attendant effector functions by providing an intact Fc-region. Thus, the antibodies created by the methods taught herein or having the property of binding to enzymatically created antibody fragments in vivo may be useful as therapeutic molecules. The anti-IgG cleavage fragment antibodies of the present invention can be used to treat patients in which a disease characterized by disease induced proteolytic cleavage of IgG. In one aspect, the anti-IgG cleavage fragment antibodies may be used to restore effector functions to antibody fragments which retain target specific binding capability.

While having described the invention in general terms, the embodiments of the invention will be further disclosed in the following examples.

### EXAMPLE 1: CLEAVAGE ANALYSIS OF HUMAN IgG HEAVY CHAIN

Proteolysis of human IgG heavy chain by matrix metalloproteinases, cathepsins, human neutrophil elastase (HNE), and selected pathogen enzymes such as staphylococcal glutamyl endopeptidase (V8 protease), and immunoglobulin degrading enzyme of streptococcus (IdeS) was studied.

A purified monoclonal antibody comprising a human IgG heavy chain was contacted with the proteases described and sampling was conducted over various durations of contact. Fragmentation in the samples was evaluated using the Agilent micro fluidics "lab-on-a-chip" technology for in vitro biosizing (Goetz H et al. Biochemical and Biophysical Methods 60; 281-293, 2004).

Antibody Substrates. Monoclonal antibodies were either fully human, recombinant humanized murine antibodies or human/murine chimeric antibodies possessing human constant domains and hinge regions of the IgGlkappa class/subclass and species: Mab1 is a human IgG1 which binds a pathogen, Mab2 (anti-cytokine) is a human/murine chimeric IgG1 antibody possessing human constant regions and hinge domain, and Mab3 is a CDR-grafted humanized IgG1. All of the antibodies contain a kappa light chain.

Proteolytic Enzymes and Methods of Testing. Human pro-MMP-2, MMP-7 and pro-MMP-9 were obtained from Chemicon International (Temecula, CA) and were activated by incubation with 1 mM *p*-aminophenylmercuric acetate (APMA; CalBiochem, San Diego, CA) for 16 hr at 37°C prior to use (Marcy et al., 1991). Recombinant human active MMP-12 was obtained from R&D Systems. Recombinant MMP-1 was a generous gift from Dr. Hideaki Nagase. Human pro-MMP-3 was transiently expressed in HEK cells with a histidine tag in place of the hinge and hemopexin domains. The pro-MMP-3 variant was activated by incubation at 55 °C for 25 minutes as described (Koklitis et al., 1991). Cathepsins B, D, G, S and proteinase 3 were obtained from Athens Research & Technology (Athens, GA). The coagulation enzymes thrombin, F.Xa, F.IXa, F.XIIa and kallekrein, as well as plasmin and plasminogen, were purchased from Enzyme Research Laboratories (South Bend, IN). Tissue plasminogen activator (Activase) was a product of Genentech (South San Francisco, CA). Streptokinase and activated protein C were obtained from Sigma (St. Louis, MO). Staphylokinase was obtained from Affinity BioReagents (Golden, CO). Staph. aureus V8 glutamyl endopeptidase I was obtained from Pierce Biotechnology (Rockville, IL). Recombinant immunoglobulin degrading enzyme of Streptococcus pyogenes (IdeS) was provided by Dr. Lars Bjorck of the University of Lund (Lund, Sweden).

Proteinase digestions of purified IgGs were carried out at pH 7.5 in phosphate-buffered saline (PBS) or, for the metalloproteinases, in Tris-buffered saline buffer at 37 °C. Calcium chloride was included in the metalloproteinase reactions at 1mM for MMP-12 and 10 mM for MMP-3; otherwise no additives were used. Antibody concentrations were typically 1 or 2 mg/mL and reactions were initiated by addition of enzyme to a 1% (w/w) ratio to IgG. Aliquots (10-20 µL) were removed at indicated times and the reactions were stopped either by adjustment to 20 mM EDTA (metalloproteinase incubations) or to 1 mM iodoacetamide (cysteine proteinases) or by rapid freezing.

The major proteolytic cleavage positions in the IgG1 hinge were identified for enzyme-generated fragments by N-terminal sequencing of the purified Fc fragment (MMP-3 and MMP-12) and/or high resolution mass spectrometric analyses of the purified Fab (neutrophil elastase, plasmin) and F(ab')₂ (cathepsin G, glutamyl endopeptidase and IdeS) fragments. Fragmentation was evaluated using the Agilent microfluidics "lab-on-a-chip" technology.

Results. A list of proteinases that were examined and the results of the analysis of the primary products of proteolytic cleavage of human IgG1 is presented in Table 1. Several enzymes did not fragment IgG1 under the conditions used. Among the active proteinases, relative specific activities under the conditions described were: IdeS > MMP-12 > MMP-3, glutamyl endopeptidase > neutrophil elastase > cathepsin G, plasmin > MMP-2, MMP-9 > MMP-7.

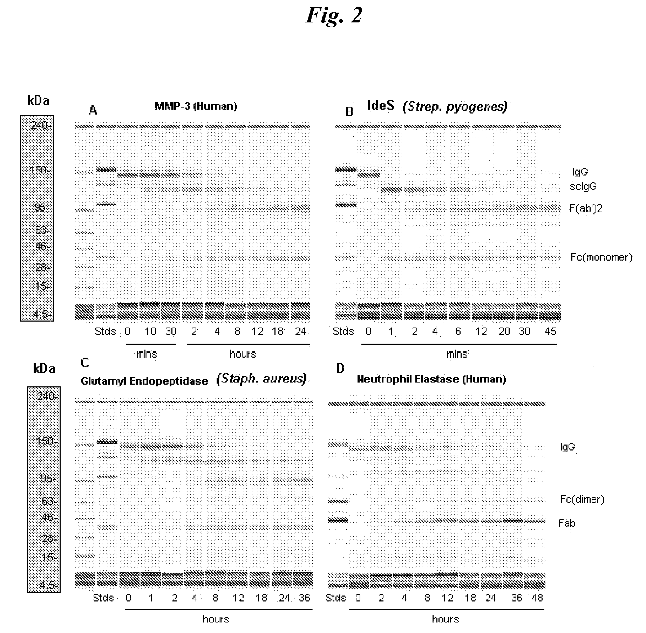

Figure 2 depicts biosizing analyses of IgG before and during proteolytic enzyme treatment. MMP-3, gluatamyl endopeptidase I and IdeS were each observed to cleave IgG1 in a stepwise fashion (Figures 2A, 2B and 2C, respectively). In each case, an early intermediate of approximately 135,000 Da was generated which was subsequently converted to a species of approximately 100,000 Da. A fragment of approximately 35 kDa, presumably the Fc monomer, was also formed during these reactions. The molecular weight (35 kDa) gauged by gel migration is larger than that predicted by the heavy chain fragment amino acid sequence which would be 211 to 215 residues (between residue 232 and 237 to the 447^{th} residue at the C-terminus of the heavy chain) but consistent with the fragment containing the glycosylation site in the CH2 domain. The disappearance of intact IgG (160,000 Da) occurred over a period of several hours with MMP-3 and glutamyl endopeptidase I, and within a minute or less with IdeS under these conditions. All digestions were carried out under comparable conditions as described.

The 135 kDa intermediate was found to result from a single proteolytic cleavage in one of the heavy chains in the lower hinge domain. Under non-denaturing conditions, the intermediate is indistinguishable from intact IgG in certain physical properties, such as migration in size exclusion chromatography (data not shown). However in SDS gels, and the present microcapillary electrophoresis system, the cleavage fragment of the Fc region (CH2-CH3 domains of the heavy chain) separates from the rest of the structure to reveal the reduced size intermediate (135 kDa). The size of this species is consistent with a singly cleaved IgG as reported by Gearing (2002 *supra*). Extended incubation of IgG1 with the three enzymes resulted in conversion of the scIgG intermediate to the F(ab')₂ fragment and Fc.

Among the enzymes exhibiting the ability to cleave IgG1 of the different monoclonal IgG1-based substrates tested, was a consistent finding that the initial cleavage to the single-cleaved intermediate was relatively rapid, and extended times were required for the second cleavage to F(ab')₂ to occur. Also shown in Figure 2D is a digest of Mab1 with human neutrophil elastase (HNE). HNE differed from the three enzymes above in that it cleaved IgG in the upper hinge to yield Fab fragment and a corresponding disulfide-linked Fc dimer.

The major proteolytic cleavage positions in the IgG1 hinge were identified for the purified Fc fragment (MMP-3 and MMP-12) and/or high resolution mass spectrometric analyses of the purified Fab (neutrophil elastase, plasmin) and F(ab')₂ (cathepsin G, glutamyl endopeptidase and IdeS) fragments. The amino acid sequence of the human IgG1 hinge region is presented in Figure 3, with the identified positions of enzymatic cleavages indicated. Extended digestion with proteinases that cleaved in the upper hinge yielded two Fab fragments; enzymes that cleaved in the lower hinge (below the core hinge disulfide bonds) yielded F(ab')₂S.

The dominant site of enzymatic cleavage were identified or confirmed for each enzyme including human MMP-3 and -12, human cathepsin G, human HNE, staphylococcal glutamyl endopeptidase I and streptococcal IdeS based on analysis of both the carboxy- and amino-terminal residue in the F(ab')₂ or Fc fragment, respectively (Table 1). Secondary cleavage sites were observed in some cases during extended incubations (e.g. cathepsin G and HNE), and it was uncertain if these are alternative cleavage sites for the indicated proteinase or the result of minor, proteinase contaminants in these enzyme preparations. The MMP-12 and HNE cleavage sites in IgG have not been previously reported. For other proteinases, the identified major IgG cleavage positions agreed with previously reported results (Chuba, 1994; Diemel et al., 2005; Gearing et al., 2002; Vincents et al., 2004; Yamaguchi et al., 1995).

The cleavage positions differ slightly among the enzymes; with proteolysis occurring after proline-245, glutamic acid-246 and glycine-249 for MMP-3, V8 and IdeS, respectively. These differences in cleavage position do not impact molecular weight as detectable using the micro-capillary electrophoretic biosizing system (Agilent Technologies). Longer incubation times with MMP-3, V8 and IdeS allow the complete conversion to F(ab')₂ fragments. The digestion of IgG1 by HNE differs from the other proteases as it cleaves before the core hinge disulfides (cysteines 238 and 241) at histidine 236 to yield a Fab product and disulfide linked Fc (see Fig. 2D).

The cleavage sites are based on EU numbering and relate to the residues shown in Fig. 3 and SEQ ID NO: 1 which encompasses from Ser²¹⁹ through Phe²⁴³ of a human IgG1 class antibody. Several proteinases cleaved IgG1 below the hinge domain, and yielded F(ab')₂ fragments of slightly different lengths (spanning Ala²³¹ to Gly²³⁷). Many of the IgG-degrading proteinases characterized in this study have been reported to be expressed, or to be abundant, at sites of inflammation (HNE, cathepsin G, MMP-12), in the tumor or wound-healing environment (MMP-2, MMP-3, MMP-7, MMP-9, plasmin), and at sites of infection (glutamyl endopeptidase, IdeS) (Dollery et al., 2003; Kilian et al., 1996; Rooijakkers et al., 2005; Schönbeck et al., 1999; Shapiro, 1999; Sukhova et al., 1998; van Kempen et al., 2006; Vincents et al., 2004)). For many cases, it is unlikely that the extracellular expression of specific proteinases is primarily directed toward host IgG; rather, their elaboration is associated with the physiology of disease (e.g. matrix metalloproteinases in the tumor environment). Nevertheless, these *in vitro*, purified enzyme/monoclonal antibody degradation studies indicated that human IgGs are not resistant to a number of proteinases with potential relevance to human disease.

For enzymes that converted IgG1 to F(ab')₂ (the majority), the cleavages were highly specific and self limiting (unlike pepsin digestion that reduces the Fc domain to small peptides). With the exception of IdeS, the rates of IgG1 cleavage with most of these extracellular proteinases were generally slower than that seen with pepsin under its optimal conditions (e.g. pH 4.0). Proteolytic fragmentation to F(ab')₂ proceeded via single-cleaved intermediates in a two-step process. The single-cleaved intermediate of IgG1 was previously proposed as a possible intermediate during digestion of IgGs with MMP-3 (Gearing et al., 2002) and with IdeS (Vincents et al., 2004). In the present studies, it was consistently observed that the first cleavage to the single-cleaved intermediate occurred relatively rapidly compared to the second, slower cleavage that yields F(ab')₂. The studies reported here focused on IgG1 because it is the predominant isotype of IgG in human circulation. A limited number of other human isotype experiments were carried out to determine relative susceptibilities to MMP-3 and IdeS. In these it was observed that IgG4 was comparable in susceptibility to IgG1, whereas IgG2 and IgG3 were more resistant under these conditions (data not shown). Comparable investigations of IgA, IgM, IgE, IgD degradation were not done.

All of the information is summarized in Table 1, where "Coagulation proteinases" included F.XIIa, FIXa, F.Xa, thrombin and activated protein C; plasmin was plasminogen co-incubated with plasminogen activators; tPA, streptokinase and staphylokinase; "plasminogen activators alone" are without plasminogen; and the MMPs were recombinant proteinases obtained either as the active form or the pro-enzyme as detailed in the Materials; and "None" denotes no detectable cleavage in 24 hours. Except where indicated all enzymes where human. The residue designations are for the EU numbering system for the IgG1 antibody heavy chain where the 25 residues of SEQ ID NO: 1 correspond to residues 219 through 243 of the complete mature heavy chain.

**Table 1.**

| Enzyme | Source | Proteinase Type | Disease Association (Ref) | Cleaved Site | Major Product |
|---|---|---|---|---|---|
| Cathepsin G | Human Neutrophil granules | Serine endopeptidase | Emphysema, IPF, RA (2,3) | Glu²³³-leu²³⁴ | F(ab')₂ + Fc |
| Cathepsin B | " | " | | | None |
| Cathepsin D | " | " | | | None |
| Neutrophil elastase (HNE, leukocyte elastase, PMN elastase) | " | " | Amyloidosis, lung emphysema, cystic fibrosis, ARDS, RA, tumor invasion (2,3) | Thr²²³-his²²⁴ | Fab + Fc |
| | neutrophils | | | | |
| Pancreatic elastase | | | Pancreatititis (3) | | |
| Proteinase 3 (myeloblastin) | " | " | | | None |
| Tryptase | " | " | Anaphylaxis, fibrosis (2) | | None |
| | mast cells | | | | |
| Chymase | " | " | Inflammation, cardiovascular diseases (2, 3) | | None |
| | mast cells | | | | |
| Kallekrein | " | " | | | None |
| Coagulation proteinases | " | " | | | None |
| Plasmin (fibrinolysin) | " | " | Cell migration (e.g.tumors)(2) Streptococcal infection (6) | Lys²²³-thr²²⁴ | Fab + Fc |
| Plasminogen activators alone | " | " | | | None |
| Interstitial collagenase (MMP-1) | Human (fibroblasts, chondrocytes , | Metalloendop eptidase | RA, OA, IBD, IPF, aneurysms (1) | | None |
| Gelatinase A (MMP-2) | " | " | Invasive tumors (1) | Glu²³³-leu²³⁴ | F(ab')₂ + Fc |
| | tumor cells, fibroblasts | | | | |
| Stromelysin (MMP-3) | " | " | RA, OA, atherosclerotic plaque, Crohn's disease, colitis, some tumors (1, 4) | Glu²³³-leu²³⁴ | F(ab')₂ + Fc |
| | fibroblasts, chondrocytes , osteoclasts, macrophages | | | | |
| Matrilysin (MMP-7) | " | " | Invasive tumors (1, 4) | Leu²³⁴-leu²³⁵ | F(ab')₂ + Fc |
| | glandular epithelial cells | | | | |
| Collagenase 2 (MMP-8) | " | | Inflammation, RA, OA (1, 4) | | None |
| | neutrophils | | | | |
| Gelatinase B (MMP-9) | " | " | Inflammation, aortic aneurysms, ARDS, bums RA > OA, inflammatory cell tumor infiltrates (1, 4) | Leu²³⁴-leu²³⁵ | F(ab')₂ + Fc |
| | normal and tumor cells, activated monocytes, neutrophils, T cells | | | | |
| Macrophage metalloelastase (MMP-12) | " | " | Inflammation, tissue destruction when over-expressed, aneurysms, atherosclerotic plaque (1) | Pro²³²-glu²³³ | F(ab')₂ + Fc |
| | macrophages | | | | |
| Cathepsin S | " | Cysteine endopeptidase | | | None |
| Glutamyl endopeptidase I (Glu V8 protease) | *Staph. aureus* | Serine endopeptidase | *Staph. Aureus* infection (2) | Glu²³³-leu²³⁴ | F(ab')₂ + Fc |
| Immunoglobulin degrading Enzyme of Streptococcus (IdeS) | *Strep. Pyogenes* | Serine endopeptidase | *Strep. Pyogenes* infection (5) | Gly²³⁶-gly²³⁷ | F(ab')₂ + Fc |

| | | | | | |
|---|---|---|---|---|---|
| (1) Barrett A. J., Rawlings N. D. and Woessner J. F.(Eds.), Handbook of Proteolytic Enzymes Vol. 1, Elsevier, Amsterdam, 2004. (2) Barrett A. J., Rawlings N. D. and Woessner J. F.(Eds.), Handbook of Proteolytic Enzymes Vol. 2, Elsevier, Amsterdam, 2004. (3) Powers, JC.. "Proteolytic Enzymes and Disease Treatment" 1982. In: Feeney and Whitaker (eds). Modification of Proteins: Food, Nutritional, and Pharmacological Aspects. Advances in Chemistry Series 198. ACS, Washington, D.C. 1982 pp 347-367. (4) Tchetverikov I, Ronday H. K., van El B., Kiers G. H., Verzijl N., TeKoppele J. M., Huizinga T. W. J., DeGroot J. and Hannemaaijer R., 2004. MMP Profile in paired serum and synovial fluid samples of patients with rheumatoid arthritis. Ann.Rheum.Dis. 63, 881-883. (5) Vincents B., von Pawel-Rammingen U., Björck L. and Abrahamson M., 2004. Enzymatic characterization of the streptococcal endopeptidase, IdeS, reveals that it is a cysteine protease with strict specificity for IgG cleavage due to exosite binding. Biochemistry 43, 15540-15549. (6) Sun H., Ringdahl U., Homeister J.W., Fay W.P., Engleberg N.C., Yang A.Y., Rozek L.S., Wang X., Sjobring U., Ginsburg D., 2004. Plasminogen is a critical host pathogenicity factor for group A streptococcal infection. Science. 305, 1283-1286. | | | | | |

### EXAMPLE 2: CLEAVAGE OF IgG IN AN INFLAMMATORY EXUDATE

Inflammatory exudates and other such fluids are expected to possess proteolytic enzymes associated with the inflammation and wound healing. For this purpose, samples of wound fluid were obtained from Ethicon Inc.

First, an antibody substrate, which comprises human heavy chain constant domains was randomly biotinylated. Ten microliters of the biotinylated substrate antibody was added to 190 microL of the wound fluid and incubated at 37°C for 8-24 hours. At specified times, samples were removed. The starting IgG and samples from the various times were applied in separate wells to a 4-12% Bis-Tris gel and subjected to SDS PAGE. The separated bands were transferred to a nitrocellulose membrane and, following blocking with 0.1M Tris buffered saline containing 0.1% Tween 20 and 10% blocking grade milk ("Blotto"), the blot was developed using AVIDIN-D -horseradish peroxidase reagent followed by TMB (membrane) substrate.

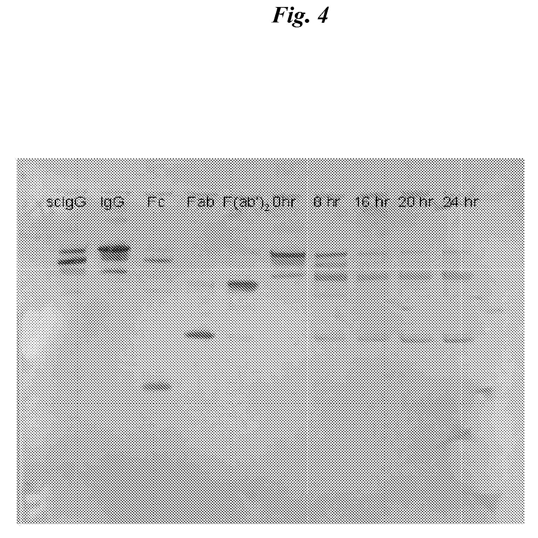

As evidenced by the gel image shown in Figure 4, there was a loss of intact IgG by 8 hr and the appearance of bands similar in size to the F(ab')2 and Fab standards. The results of this experiment indicate that proteolysis of IgG by enzymes in an inflammatory fluid occurs over a several hour period and yields fragments that correspond with fragments produced by in vitro proteolysis with purified enzymes.

### EXAMPLE 3: PREPARATION OF REAGENT

The determination of the presence of host (patient) antibody fragments produced by endogenous proteases requires a reagent which selectively binds to the cleaved IgG but not intact IgG. Both identification of the cleaved component and a quantitative difference between fragment content in samples from patients with disease as compared to the normal population should be able to be assessed using the reagent.

The detection of unknown, but likely small amounts of IgG fragments in solutions containing relatively high concentrations of intact IgG is difficult. Although scIgG has been noted as a possible IgG cleavage fragment (Gearing 2002 supra), quantitation in human samples has not been previously performed. For this purpose, a reagents with the necessary specificity were generated in rabbits having with a high degree of specificity for cleaved but not intact IgG.

Three conjugated, and progressively longer single-chain peptide analogs of the human IgG1 hinge were used for immunization (at Invitrogen Corporation). An 8-mer peptide corresponding to the sequence of amino acids on the amino terminal side of the MMP-3 cleavage site was covalently attached to keyhole limpet hemocyanin (KLH) via the N-terminus (TCPPCPAP, residues 7-14 of SEQ ID NO: 1). Extended peptides corresponding to the glutamyl endopeptidase site (TCPPCPAPE, residues 7-15 of SEQ ID NO: 1) and the IdeS site (TCPPCPAPELLG, residues 7-18 of SEQ ID NO: 1) were separately prepared as immunogens. New Zealand rabbits (two per immunogen) were immunized by subcutaneous injection of 0.2 mg conjugated peptide in complete Freund's adjuvant and re-boosted three additional times with 0.1 mg antigen in incomplete Freund's adjuvant on days 14, 42 and 56. Serum was collected at 4, 8 and 10 weeks and pooled per immunogen for antibody purification. The immune titers were monitored by an ELISA based on solid phase antigen peptide.

Affinity purification of antibodies employed the respective peptide antigens immobilized on an activated support. The antiserum from the two rabbits immunized with the same antigen was pooled and passed through the antigen column after which the column was extensively washed. Specific antibodies were eluted as low affinity and high affinity pools using 3M KSCN and 0.1M glycine, pH 2.5, respectively. The two pools yielded indistinguishable binding characteristics and were used interchangeably and/or pooled. The three separately eluted pools of bound antibodies were next subjected to a second affinity adsorption step, this time on a column containing an intact antibody comprising human IgG1 heavy chain constant regions (Mab3). The intent of the second affinity chromatography step was to remove undesirable antibodies that might recognize intact IgG. However, little or no rabbit antibody was adsorbed to the IgG column suggesting that the population of antibodies was reactive only with the "cleaved" sequence with its exposed carboxy terminus.

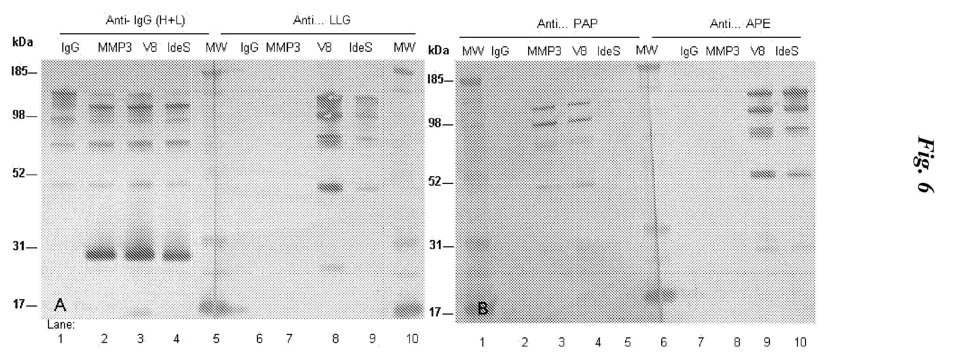

The individual affinity-purified rabbit anti-peptide antibodies were tested for their ability to bind to enzymatically-generated fragments of human IgG as well as intact IgG by ELISA (Fig. 5). The purified antibodies from the rabbits immunized with KLH conjugated to residues 7-14 of SEQ ID NO: 1 (the MMP-3 site analog) did not bind intact IgG and were highly specific for scIgG and F(ab')₂ produced by digestion of IgG with MMP-3. This antibody preparation showed minimal reactivity to scIgG and F(ab')₂ produced with V8 protease or IdeS. In contrast, the antibodies obtained from rabbits immunized with the V8-cleavage site hinge peptide analog (residues 7-15 of SEQ ID NO: 1) and the IdeS-cleavage site hinge peptide analog (residues 7-18 of SEQ ID NO: 1) showed a cross-reactive binding profile for scIgGs and F(ab')₂ produced by either of these two enzymes. However, these preparations showed minimal reactivity for the MMP-3 digested products. None of the antibody preparations bound to intact IgG and none of the antibody preparations was comparably reactive with fragments, including F(ab')₂ and scIgG, produced by three different enzymes as shown in Figure 6.

The intended use of the anti-hinge reagent is the detection of scIgGs and F(ab')₂ (and other potential fragments) that are produced in complex in vivo settings by enzymes present in disease specific tissues or produced by disease specific cell types or cell populations, e.g. infiltrating macrophages or neutrophils. For optimal coverage of potential IgG fragments, it was considered preferable to have as broad a profile of cleavage site recognition as possible. For this reason, a mixture of each of the three rabbit antibody pools was prepared at 0.33 mg/mL of each component (total = 1 mg/mL) for use in subsequent Western blotting and serum-based ELISA tests. This pooled reagent is referred to as RAH-1.

### EXAMPLE 4: SINGLE CHAIN CLEAVED IMMUOGLOBULIN ASSAY

The novel assay employed for detecting scIgG in serum using, as a capture reagent, the RAH-1 capable of binding cleaved human IgG but not intact human IgG is described in detail as follows.

Using a Chemiluminescence ELISA, regions of a Nunc Chemiluminescence 96-well plates were coated with RAH-1 at 10 mg/ml in PBS. The rest of the plate was left un-coated (1X PBS alone). The plates were incubated at 4°C over night. Plates were washed and 200 ml/well of Chemicon International's ChemiBLOCKER (C# 2160) was added to the plates and incubated at 37°C for 30 minutes. The blocking buffer was aspirated from the wells and the standards and samples were added to the plate. The standard Mab3, scIgG digested with V8, was added in duplicate starting at 50 mg/ml in PBS containing 1%Casein and 3%BSA using serial four-fold dilutions. The disease serum samples were added at a 1:50 dilution in the same buffer. Plates were washed and a 1:6000 dilution of Jackson Immuno Research's HRP conjugated AffiniPure F(ab')₂ Fragment Donkey anti-human IgG (H+L) which has minimal cross reactivity to various animals including rabbit was added to all wells. This was added in a dilution of PBS with 1%Casein and 3%BSA and incubated at 37°C for 1 hour. The plates were washed thoroughly and 100 ml/well of HRP substrate (Roche's BM Chemiluminescence POD, 582 950) was added seconds before plate was read on the luminescent reader.

The average luminescence from the 0 ng/ml scIgG wells on the standard curve was subtracted from all wells that were exposed to the RAH-1 coat. This subtraction controls for any non-specific reactivity of the secondary with the RAH-1. Then, the value for each donor on non-RAH coated wells was subtracted from the previously-adjusted value of the RAH coated wells. This accounts for any non-specific reactivity in the serum to the plate.

### EXAMPLE 5: USE OF REAGENT TO DETECT DISEASE ASSOCIATED PROTEOLYTIC ACTIVITY

The RAH-1 reagent was tested for its ability to detect IgG fragments in another inflammatory fluid, the synovial fluid of a patient with rheumatoid arthritis (RA).

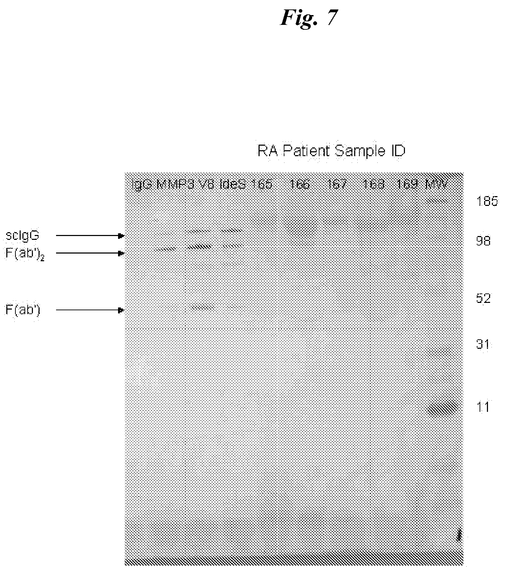

A collection of synovial fluid samples from RA patients were purchased commercially from Bioreclamation. Samples were diluted 1:5 in LDS sample buffer and 10 mciroL of each sample loaded onto a 4-12% Bis-Tris gel. As a control for the reactivity of RAH-1, intact IgG (Mab3), or protease digested IgG (partial digests of Mab3 with MMP-3, glutamyl endopeptidase and IdeS) was loaded onto the gel as well. Following SDS PAGE, the gel was transferred to nitrocellulose membrane and blocked with Blotto. The membrane was then incubated with a 1:2,500 dilution of the RAH-1 in blotto, washed with 0.1M tris buffered saline, pH 7.5 containing 0.1% Tween 20 and incubated with a 1:5,000 dilution of goat anti-rabbit IgG (H&L) horseradish peroxidase conjugate. The blot was then developed using TMB membrane. As shown in Figure 7, the RAH-1 preparation did not react with the intact IgG , but detected scIgG, F(ab')₂ possibly Fab' from all 3 protease digests. For all five synovial fluid samples from RA patients, a band was detected at the approximate size of scIgG, F(ab')₂ and Fab', suggesting that these proteolytic fragments were present within the synovial fluid from individuals with RA.

### EXAMPLE 6: USE OF REAGENT TO MONITOR DISEASE

Blood plasma or serum is a more convenient for biomarker testing than biological fluids or tissue extracts, such as synovial fluid. However, the advantage of synovial fluid is that it is a self-contained and local environment in which the proteases are active and in which the IgG fragments might be expected to accumulate as described in Example 2. Nevertheless, the ease and prevalence of serum for testing makes it a considerably more likely sample tissue for biomarkers, including IgG breakdown products.

Before initiating testing for IgG fragments in different types and sources of serum, it was desirable to establish which if any of the proteolyzed IgG fragments would circulate for a sufficient period to allow its accumulation and quantification. To answer this question, a comparative pharmacokinetic study was designed. The following PK experiment in mice was modeled on several similar previously reported studies in which human IgGs generally exhibit terminal half-lives of 10-20 days.

Fractionated proteolysis products, Mab2 IgG1, and the scIgG and F(ab')₂ generated with MMP-3, were prepared as follows. A 20 milligram quantity of Mab2 IgG was digested with heat-activated MMP-3 as described in Example 1. The digestion was initiated by addition of enzyme to a 4 mg/mL solution of Mab2 in tris-buffered saline containing 10mM CaCl₂, pH 7.5 at 37°C. The reaction was terminated by the addition of EDTA to a final concentration of 20mM at 48 hours. There was no residual intact IgG and the percent of scIgG, F(ab)₂ and Fc was 24%, 41% and 36%, respectively, based on Agilent bio-sizing analysis (8862-67). The terminated digest was subjected to a two-step purification to remove the Fc fragment and to separate purified scIgG and F(ab)₂. In the first step, the digest was subjected to chromatography using protein A-Sepharose. The unbound material from the column contained the F(ab)₂ fragment and no detectable intact IgG or scIgG. Treatment of the column with 0.1M sodium citrate, pH 3.5, resulted in the elution of a mixture of Fc-containing components, the Fc fragment and scIgG. The fractions were immediately neutralized to pH 7 by the addition of 1/10^{th} volume of 2M Tris, pH 7.0. The neutralized material was concentrated to approximately 1mL and dialyzed into phosphate-buffered saline, pH 7.5. The Fc fragment was separated from scIgG by size exclusion chromatography on Superdex 200 (column volume = 100mL). Two peaks eluted from the column, which were subsequently identified using the Agilent biosizing technique previously described as a 135 kDa conforming to the gel band position of scIgG and a lower molecular weight peak identified as the Fc monomer fragment of approximately 35 kDa. The purified scIgG and F(ab)₂ components were contacted with ActicleanEtox (0.5mL of gel per 5 mL of each protein solution) to reduce endotoxin to AALAC specifications for allowable intravenous injection in mice (<40 EU/kg).

For this study, the equivalent milligram amount (1.9 mg/kg) of intact mouse-human chimeric monoclonal antibody, Mab2 IgG1, and the scIgG and F(ab')2 generated with MMP-3 was tested as described below.

A group of twenty-one female Balb/c mice (Ace Animals) were used for the pK study. Terminal bleeds were taken via cardiac puncture from three randomly selected mice prior to the experiment to serve as baseline controls. The remaining eighteen female Balb/c mice were weighed and placed into six equal groups. Two groups were injected with intact Mab2 IgG1, two groups with Mab2 scIgG1 produced with MMP-3 and two groups with Mab2 F(ab')2 produced with MMP-3. All injections were i.p. at a constant dose volume of 10 ml/kg based on individual animal weight at 0.19 mg/ml. Therefore, each animal received a 1.9 mg/kg dose at day zero. Approximately 80 ul of blood was collected at 1 h, 24 h, 7 d, 21 d, 35 d from the first of the two groups and at 5 h, 48 h, 14 d and 28 d from the second group The serum samples were stored at 20°C until tested.

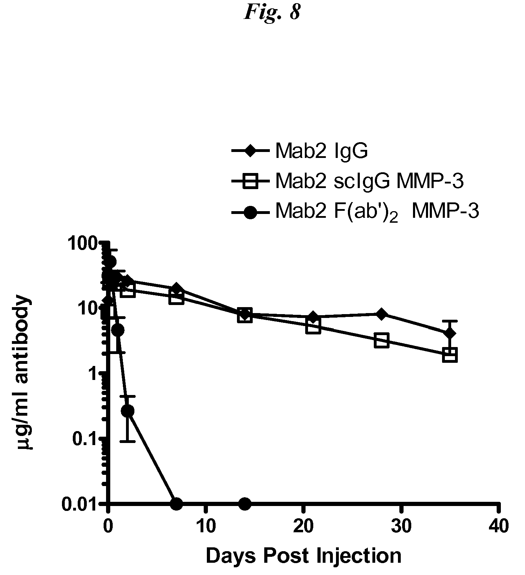

The IgG and IgG fragment concentrations of the collected serum was quantified via enzyme-linked immunosorbent assay (ELISA). A 0.5 ug/ml dilution in PBS of Jackson Immuno-research: Goat Anti-Human IgG, F(ab')2 fragment specific (with minimal cross-reactivity to bovine, horse and mouse serum proteins) was used to coat Costar 3369 plates. Plates were blocked with PBS/casein/BSA. Following blocking, standards and samples were added in a PBS/1%casein/3%BSA. Standards included serial dilutions of the following: a murine/human IgG1, murine/human scIgG1 MMP-3 or murine/human F(ab')2 MMP-3 starting at 1000 ng/ml Each time point sample was serially diluted from 1:10 to 1:163,840 in PBS/1%casein/3%BSA. Human IgGs bound to the plates coated with anti-human capture antibody were detected with 50ul/well Jackson Immuno-research: Goat Anti-Human IgG (H+L) (with minimal cross-reactivity to bovine, horse and mouse serum proteins; 109-035-083) and incubate for one hour at RT. The plates were thoroughly washed and exposed to 50ul/well *O*-phenylenediamine substrate for approximately 10 mins and stopped with 50 ul/well 3M HCL and read at 490-650nm. The results are shown in Figure 8.

The results of the mouse PK experiment indicate that the scIgG possesses an extended circulating lifetime, but that the F(ab')₂ does not. The very rapid clearance of F(ab')₂ in the mice is consistent with the rapid disappearance of this fragment in humans (Roskos LK et al. Drug Dev. Res. 61: 108-120, 2004). These results point to scIgG as the most abundant, long-lived, and useful proteolytic component of IgG for biomarker purposes.

### EXAMPLE 7: PROTEOLYTIC ENZYMES IN DISEASE

In order for the scIgG to be a useful disease biomarker, it must exhibit differential quantity in samples obtained from patients in defined disease categories as compared to healthy people.

A commercial source of serum from diseased individuals was identified as Genomics Collaborative (now SeraCare Life Sciences Inc.). Small volumes (300 microL) of serum from 10 different individuals within each of 8 diseases were purchased. The disease categories were rheumatoid arthritis, osteoarthritis, asthma, type-1 diabetes, breast cancer, lung cancer, myocardial infarction, and congestive heart failure. In addition, serum from 28 age-matched and gender-matched normal healthy volunteers were obtained from this vendor as controls.

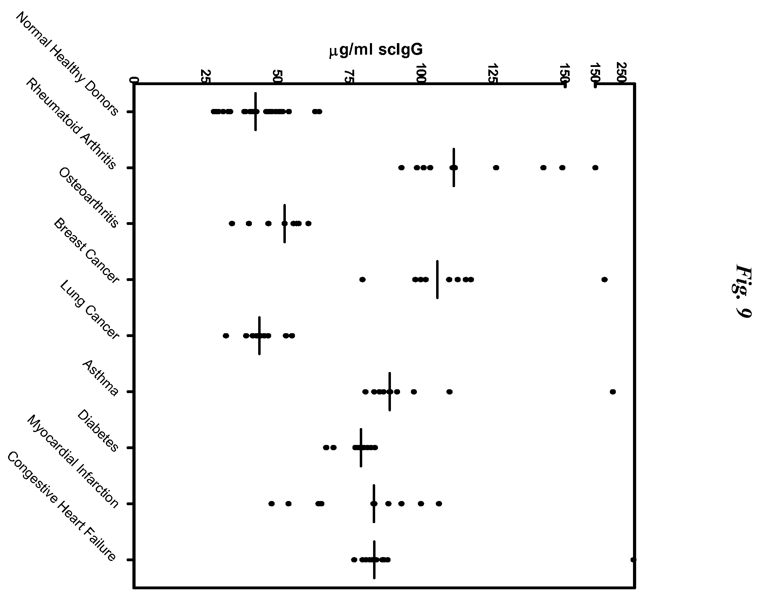

Using the assay as described in Example 4, the samples were analyzed and the results shown in Fig. 9. The assay, based on the selectivity of the RAH-1 reagent, demonstrated that IgG cleavage products comparable to those generated by known specific proteases are clearly detectable and above levels maintained in healthy or normal donors for an inflammatory autoimmune disease, rheumatoid arthritis. In contrast, patients with osteoarthritis showed levels which were similar and in the range of those for the normal individual's samples.

### EXAMPLE 8: MODIFIED SINGLE CHAIN CLEAVED IgG ASSAY

A solid phase assay, ELISA, using reagent RAH-1 for detection of scIgG in serum was described in Example 4. In order to optimize the detection range for scIgG concentrations serum samples specific changes were made.

The plates used were Immulon 4 HBX plates (VWR) coated with rabbit polyclonal antibodies (RAH-1) at a concentration of 5 µg/mL in PBS pH 7.2 (100 µL per well) by sealing and incubating the plate for 1 hour at room temperature. Thereafter, the plate is washed 3X with PBS, 0.05% Tween (Sigma) on automatic plate washer. All samples and standards are diluted with PBS containing 1%BSA, 0.05% Tween. Anti-IgG Fc-Biotin (USBIologicals, Swampscott, MA) is the means of detection of scIgG standards or scIgG unknowns in serum dilutions.

The plate is blocked using 200 µL of SuperBlock (Pierce) for 15 minutes at room temperature (RT) with shaking and then washed plate 3X with PBS, 0.05% Tween on an automatic plate washer.

The standard material; Mab1 protease digestion product, is added to duplicate wells starting at 600 ng/mL (100 µL per well, 3 fold dilutions). Serum samples are diluted 1:100, 1:200, 1:400, etc. as appropriate). Samples are added in duplicate at the same time and incubated for one hour at RT on a shaker followed by 3X washing with PBS, 0.05% Tween on an automatic plate washer.

The IgG Fc Biotin dilution of 1:20,000 (dilute appropriately in assay buffer), is added to all wells (100 µL per well and incubated for one hour at RT on a shaker followed by 3X washing with PBS, 0.05% Tween on an automatic plate washer.

Add SA-HRP (Streptavidin conjugated to horseradish peroxidase, Sigma, used at a 1:30,000 dilution in PBS, 0.05% Tween, 1% BSA) is added to all wells (100 µL per well and incubated for one hour at RT on a shaker followed by 3X washing with PBS, 0.05% Tween on an automatic plate washer.

Finally, 100 µL of TMB (3,3',5,5'-Tetramethylbenzidine a peroxidase substrate) as provided by the manufacturer (Sigma) is added to each well and allowed to incubate for about 10 minutes for color development. The reaction is stopped with 75 µL of 1 N H₂SO4 and read plate at 450 nm.

Using the above ELISA format, the assay demonstrated greatly improved linearity and spike recoveries of scIgG in normal, healthy serum. The dilution linearity of the assay was determined in two serum pools, diluted 1:100 then spiked with Mab1 at concentrations of 150 ng/mL and 300 ng/mL, and further diluted to concentrations of 0, 25, 75, and 100% serum. Each sample dilution was assayed in triplicate and mean analyte recoveries were calculated. Linearity was assessed by calculation of an R2 correlation coefficient from a plot of the observed (y-axis) versus expected (x-axis) analyte recovery results for each sample pool. The R2 values were: Sample 1 Low 0.9983; Sample 1 High 0.9913; Sample 2 Low 0.9852; Sample 2 High 0.973; and dilution linearity was 100% for all dilutions.

### EXAMPLE 9: DETECTION OF SINGLE CHAIN CLEAVED IgG IN SERUM

Serum from RA patients was used exclusively in order to further study the results in Example 4 where some serum samples from this group of patients had notably higher scIgG compared to controls.

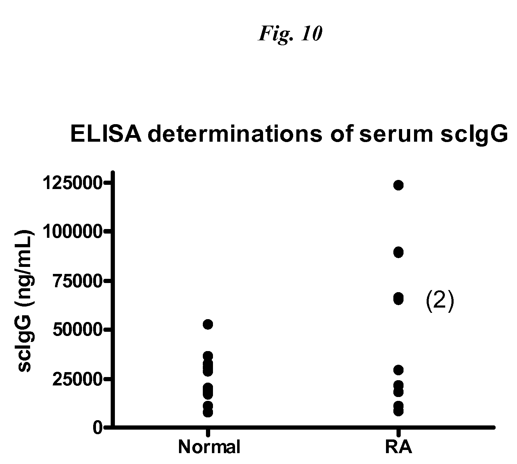

Serum samples from 10 subjects with rheumatoid arthritis (RA) and from 10 age- and gender-matched healthy individuals were obtained from Genomics Collaborative. Using the modified assay described in Example 8, the samples were analyzed and the results shown in Fig. 10. The results indicated that 4 of the 10 subjects with RA demonstrated serum scIgG concentrations >60µg/mL. In the healthy control group, scIgG concentrations ranged from <8.2µg/mL to 52.7 µg/mL. The samples for this comparison were not rigorously selected for stage of disease, treatment regimens, etc. Thus, it can be anticipated that further discrimination of healthy and disease-related serum scIgG might occur in longitudinal analyses of patients from well-controlled and prospectively designed clinical trials. However, the present assays on these commercial samples suggest that elevated scIgG concentrations can be detected in patients with disease.

### EXAMPLE 10: PREPARATION OF ANTI-HINGE IgG MONOCLONAL ANTIBODY

It would be desirable to produce a defined molecule, such as a monoclonal antibody, for manufacture and potential use in human patients which binds cleaved IgG and not intact IgG. The following procedure represents a method for generation of such a molecule.

A 12-mer peptide analog of the human IgG1 lower hinge and adjoining CH2 domain was the immunogen: TCPPCPAPELLG (residues 7-18 of SEQ ID NO: 1). The naturally occurring cysteines were replaced by alanines to give the variant TAPPAPAPELLG. An N-terminal cysteine was added to allow for conjugation to keyhole limpet hemocyanin (KLH) by standard chemical methods for reaction to free sulhydryls. The final immunogen was KLH-CTAPPAPAPELLG.

New Zealand white rabbits (3) were immunized with 0.5 mg KLH peptide in complete Freund's adjuvant using multiple subcutaneous sites (5). The animals were boosted with the 0.25 mg immunogen in incomplete Freund's adjuvant at three-week intervals for a total of 4 additional immunizations.

The serum antibody titers to a BSA-conjugated version of the same peptide were monitored during the course of the immunization by standard ELISA methods. Animals (2) were chosen for splenectomy based on the titer data. Rabbit hybridomas were generated from spleen-derived lymphocytes fused with a rabbit fusion partner cells (Spieker-Polet, 1995 PNAS USA 92: 9348 - 9352). Cell growth was examined 2-3 weeks after fusion in multiple plates.

Positive hybridomas were screened via ELISA on plates coated with the BSA-immunogen peptide conjugate. Multiple positive clones from each fusion were identified. Further screening involved binding to intact IgG1 and various enzymatically-generated F(ab')2 fragments of IgG1. From these screening and counter-screening tactics, 3 clones were chosen based on strong selectivity of binding to the immunogen peptide and to F(ab')2 fragments with termini at or near the terminus of the immunogen peptide and with minimal binding to intact IgG1. The positive hybridomas were subcloned and expanded.

Rabbit IgG was purified from individual cell supernatants by standard methods including chromatography on immobilized protein A. The specificity of the purified rabbit IgGs for binding to peptide analogs of the human IgG1 hinge, as well as intact IgG and purified IgG F(ab')2 fragments created using single or doubly cleaved Mabs using IdeS and MMP-3 enzymes were tested in standard ELISA protocols. Briefly, the peptides which were synthesized by standard peptide chemistry and were N-terminally biotinylated were captured on streptavidin-coated wells. The IgG and fragments were directly coated at 10 µg/mL. Binding of rabbit mAbs was detected by well-characterized goat anti-rabbit IgG Fc-horseradish peroxidase and OPD substrate systems.

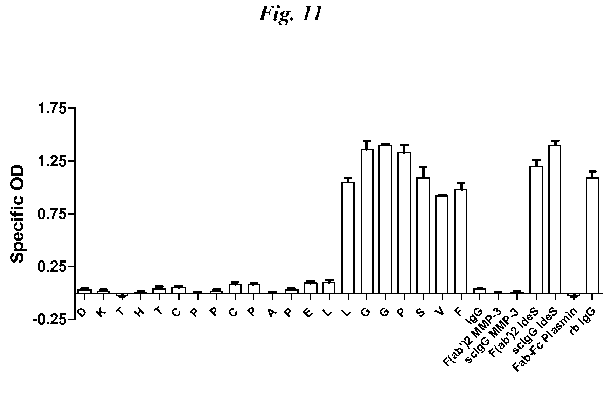

ELISA results for rabbit mAb 91-2 are shown in Fig. 11. There was a clear selectivity of binding for lower hinge peptides terminating at residues 16-22 of SEQ ID NO: 1 (L-L-G-G-P-S-V-F). There is little or no binding to the upstream residues corresponding to those segments of the upper hinge, core hinge or early lower hinge encompassed by 3-16 of SEQ ID NO: 1 (D-K-T-H-T-C-P-P-C-P-A-P-E-L-). There was negligible binding to the MMP-3 generated F(ab')2 fragment and scIgG fragment (in agreement with the lack of binding to the peptide analogs of the MMP-3 cleavage site at between residues 14 and 15 of SEQ ID NO: 1 (P-A-P*E-L-L). In contrast, there was substantial binding to the Ides-generated F(ab')2 fragment and scIgG. Thus, the rabbit mAb binding specificity conformed well to the immunogen to which it was elicited. Directly coated rb (rabbit) IgG was a positive control.

### Complement assay.

WIL2-S cells, a lymphoblastoid B-cell line expressing CD20 (ATCC), were used as target cells for CDC assays. 50µl of cells were added to the wells of 96-well plates for a final concentration of 8 X 10⁴ cells per well in RPMI, 5% heat-inactivated FBS, 0.1mM nonessential amino acids, 1mM sodium pyruvate, penicillin (500 U/ml), streptomycin (500 U/ml), 2mM L-glutamine. An additional 50µl was added to the wells with or without antibodies of various concentrations and the plates were incubated at room temperature for 2 hours. 50µl of 10% rabbit complement (Invitrogen) was added to the wells and the plates were incubated for 20 minutes at 37°C. All samples were performed in triplicate. The plates were centrifuged at 200g for 3 minutes and 50µl of supernatant was removed to separate plates and CDC was measured with LDH cytotoxicity detection kit (Roche). Absorbance was measured using a Spectra max Plus 384 (PerkinElmer). Data were normalized to maximal cytotoxicity with Triton X-100 (Sigma Aldrich) and minimal control containing only cells and complement alone.

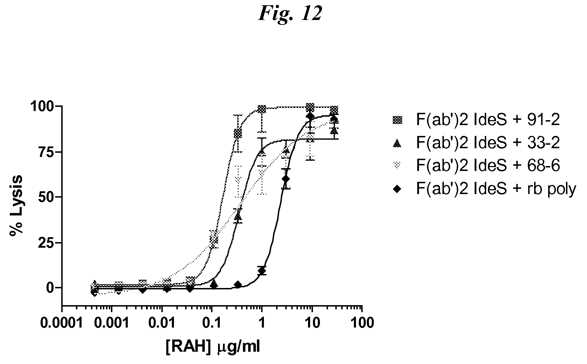

Figure 12 shows that the 3 rabbit anti-hinge mAbs were able to restore complement dependent cell lysis to the target cells when titrated in the presence of a fixed concentration of the F(ab')2 fragment of an antibody that binds CD20. The rabbit mAbs were more effective, and, at lower concentrations than a polyclonal rabbit anti-hinge mAb preparation (a component of the same detection system for serum scIgG described earlier). Intact antibody to CD20 was active, as expected, but its F(ab')2 fragment and scIgG version were not active alone. The rabbit anti-hinge mAbs were not able to direct cell lysis in the absence of cell-binding F(ab')2 fragment. These results establish that monoclonal anti-hinge antibodies can reconstitute complement-mediated effector function to otherwise inactive proteolytic cleavage products of IgG1.

## Claims

1. An antibody composition that comprises at least one antibody that specifically binds to an IgG protease cleavage product **characterized by**, a) having molecular weight which is comparable to an intact mammalian IgG under non-denaturing conditions and b) being separable into two fragments which comprise an antigen binding fragment of 135 kDa and a CH2-containing fragment under denaturing but non-reducing conditions and c) wherein the reagent does not react with intact IgG,
and wherein said antibody specifically binds to a protease specific cleavage site in human IgG1 produced by IdeS, TCPPCPAPELLG.

2. The antibody composition of claim 1, wherein said antibody is:
i) a polyclonal antiserum; or
ii) at least one monoclonal antibody.

3. An human IgG protease cleavage site peptide analog consisting of TCPPCPAPELLG covalently attached to keyhole limpet hemocyanin via the N-terminus.

4. A method of preparing of the antibody composition of claim 1 using a non-human animal immunized with the peptide analog according to claim 3, wherein the reagent is purified from the serum of said animal by pre-absorption on a human IgG affinity matrix.

5. A method of detecting a disease process in a subject by analysis of a tissue sample from the subject, wherein said method comprises the detection by the antibody composition of claim 1 of an IgG proteolytically cleaved by IdeS **characterized by** a) having a molecular weight which is comparable to an intact mammalian IgG under physiological conditions and b) being separable into two fragments which comprise an antigen binding fragment of 135 kDa and a CH2-containing fragment under denaturing but non-reducing conditions.

6. The method of claim 5, wherein the disease is selected from an arthritic disease, a malignant disease, an infectious disease, and a vascular disease, such wherein the disease is rheumatoid arthritis, for example wherein the disease is rheumatoid arthritis and the sample is synovial fluid.

7. The method of claim 5, wherein the sample is blood or a fractionation product thereof.

8. The method of claim 5, wherein the detection is performed on tissue samples other than a blood fraction.

9. The method of claim 5, wherein the detection procedure is selected from the group consisting of ELISA, immunohistochemical staining, and Western blotting.

10. A kit including a reagent for detection of a disease marker in tissue of a subject, which reagent comprises at least one antibody that specifically binds to a cleaved IgG, which antibody is capable of detecting an IgG cleavage product **characterized by** a) having molecular weight which is comparable to an intact mammalian IgG under non-denaturing conditions and b) being separable into two fragments which comprise an antigen binding fragment of 135 kDa and a CH2-containing fragment under denaturing but non-reducing conditions and c) wherein the reagent does not react with intact IgG,
and wherein said antibody specifically binds to a protease specific cleavage site in human IgG1 produced by IdeS, TCPPCPAPELLG.

11. The antibody composition of claim 1 or claim 2 for use in therapy.

## Patentansprüche

1. Antikörperzusammensetzung, die wenigstens einen Antikörper umfasst, der spezifisch an ein IgG-Proteasespaltprodukt bindet, das **dadurch gekennzeichnet ist, dass** es a) ein Molekulargewicht aufweist, das mit einem intakten Säuger-IgG unter nicht denaturierenden Bedingungen vergleichbar ist, und b) in zwei Fragmente, die ein antigenbindendes Fragment von 135 kDa und ein CH2 enthaltendes Fragment umfassen, unter denaturierenden, aber nicht reduzierenden Bedingungen getrennt werden kann, und c) wobei das Reagens nicht mit intaktem IgG reagiert,
und wobei der Antikörper spezifisch an eine proteasespezifische Spaltstelle in menschlichem IgG1, erzeugt durch IdeS, TCPPCPAPELLG, bindet.

2. Antikörperzusammensetzung nach Anspruch 1, wobei es sich bei dem Antikörper um:
i) ein polyklonales Antiserum; oder
ii) wenigstens einen monoklonalen Antikörper handelt.

3. Menschliches-IgG-Proteasespaltstelle-Peptidanalog, bestehend aus TCPPCPAPELLG, kovalent gebunden an KLH (Keyhole Limpet Hemocyanin) über den N-Terminus.

4. Verfahren zur Herstellung der Antikörperzusammensetzung nach Anspruch 1 unter Verwendung eines mit dem Peptidanalog gemäß Anspruch 3 immunisierten nichtmenschlichen Tiers, wobei das Reagens aus dem Serum des Tiers durch Vorabsorption an einer Menschliches-IgG-Affinitätsmatrix aufgereinigt wird.

5. Verfahren zum Nachweisen eines Krankheitsprozesses bei einem Individuum anhand einer Analyse einer Gewebeprobe von dem Individuum, wobei das Verfahren den Nachweis eines durch IdeS proteolytisch gespaltenen IgG, das **dadurch gekennzeichnet ist, dass** es a) ein Molekulargewicht aufweist, das mit einem intakten Säuger-IgG unter physiologischen Bedingungen vergleichbar ist, und b) in zwei Fragmente, die ein antigenbindendes Fragment von 135 kDa und ein CH2 enthaltendes Fragment umfassen, unter denaturierenden, aber nicht reduzierenden Bedingungen getrennt werden kann, mittels der Antikörperzusammensetzung nach Anspruch 1 umfasst.

6. Verfahren nach Anspruch 5, wobei die Krankheit aus einer arthritischen Krankheit, einer bösartigen Krankheit, einer Infektionskrankheit und einer Gefäßkrankheit ausgewählt ist, etwa wobei es sich bei der Krankheit um rheumatoide Arthritis handelt, beispielsweise wobei es sich bei der Krankheit um rheumatoide Arthritis und bei der Probe um Synovia handelt.

7. Verfahren nach Anspruch 5, wobei es sich bei der Probe um Blut oder ein Fraktionierungsprodukt davon handelt.

8. Verfahren nach Anspruch 5, wobei der Nachweis an anderen Gewebeproben als einer Blutfraktion durchgeführt wird.

9. Verfahren nach Anspruch 5, wobei das Nachweisverfahren aus der aus ELISA, immunhistochemischer Färbung und Western-Blotting bestehenden Gruppe ausgewählt ist.

10. Kit, enthaltend ein Reagens zum Nachweis eines Krankheitsmarkers in Gewebe eines Individuums, wobei das Reagens wenigstens einen Antikörper umfasst, der spezifisch an ein gespaltenes IgG bindet, wobei mit dem Antikörper ein IgG-Spaltprodukt nachgewiesen werden kann, das **dadurch gekennzeichnet ist, dass** es a) ein Molekulargewicht aufweist, das mit einem intakten Säuger-IgG unter nicht denaturierenden Bedingungen vergleichbar ist, und b) in zwei Fragmente, die ein antigenbindendes Fragment von 135 kDa und ein CH2 enthaltendes Fragment umfassen, unter denaturierenden, aber nicht reduzierenden Bedingungen getrennt werden kann, und c) wobei das Reagens nicht mit intaktem IgG reagiert,
und wobei der Antikörper spezifisch an eine proteasespezifische Spaltstelle in menschlichem IgG1, erzeugt durch IdeS, TCPPCPAPELLG, bindet.

11. Antikörperzusammensetzung nach Anspruch 1 oder Anspruch 2 zur Verwendung in der Therapie.

## Revendications

1. Composition d'anticorps qui comprend au moins un anticorps qui se lie spécifiquement à un produit de clivage d'IgG par une protéase **caractérisé en ce que**, a) il a un poids moléculaire qui est comparable à un IgG de mammifère intact dans des conditions non dénaturantes et b) il est séparable en deux fragments qui comprennent un fragment de liaison d'antigène de 135 kDa et un fragment contenant CH2 dans des conditions dénaturantes mais non réductrices et c) où le réactif n'interagit pas avec un IgG intact,
et où ledit anticorps se lie spécifiquement à un site de clivage spécifique de protéase dans IgG1 humain produit par IdeS, TCPPCPAPELLG.

2. Composition d'anticorps de la revendication 1, dans laquelle ledit anticorps est :
i) un antisérum polyclonal ; ou
ii) au moins un anticorps monoclonal.

3. Analogue de peptide de site de clivage par une protéase de IgG humain constitué de TCPPCPAPELLG lié de façon covalente à l'hémocyanine de patelle par l'intermédiaire de l'extrémité N-terminale.

4. Procédé de préparation de la composition d'anticorps de la revendication 1 utilisant un animal non humain immunisé avec l'analogue de peptide selon la revendication 3, dans lequel le réactif est purifié à partir du sérum dudit animal par pré-absorption sur une matrice d'affinité de IgG humain.

5. Procédé de détection d'un processus pathologique chez un sujet par analyse d'un échantillon de tissu du sujet, où ledit procédé comprend la détection par la composition d'anticorps de la revendication 1 d'un IgG clivé de façon protéolytique par IdeS **caractérisé en ce que** a) il a un poids moléculaire qui est comparable à un IgG de mammifère intact dans des conditions physiologiques et b) il est séparable en deux fragments qui comprennent un fragment de liaison d'antigène de 135 kDa et un fragment contenant CH2 dans des conditions dénaturantes mais non réductrices.

6. Procédé de la revendication 5, dans lequel la maladie est choisie parmi une maladie arthritique, une maladie maligne, une maladie infectieuse, et une maladie vasculaire, par exemple où la maladie est la polyarthrite rhumatoïde, par exemple où la maladie est la polyarthrite rhumatoïde et l'échantillon est du liquide synovial.

7. Procédé de la revendication 5, dans lequel l'échantillon est du sang ou un produit de fractionnement de celui-ci.

8. Procédé de la revendication 5, dans lequel la détection est conduite sur des échantillons de tissu autre qu'une fraction de sang.

9. Procédé de la revendication 5, dans lequel la procédure de détection est choisie dans le groupe constitué d'un ELISA, une coloration immunohistochimique, et un transfert Western.

10. Kit comprenant un réactif pour la détection d'un marqueur de maladie dans un tissu d'un sujet, ledit réactif comprenant au moins un anticorps qui se lie spécifiquement à un IgG clivé, ledit anticorps étant capable de détecter un produit de clivage d'IgG **caractérisé en ce que** a) il a un poids moléculaire qui est comparable à un IgG de mammifère intact dans des conditions non dénaturantes et b) il est séparable en deux fragments qui comprennent un fragment de liaison d'antigène de 135 kDa et un fragment contenant CH2 dans des conditions dénaturantes mais non réductrices et c) où le réactif ne réagit pas avec un IgG intact,
et où ledit anticorps se lie spécifiquement à un site de clivage spécifique de protéase dans un IgG1 humain produit par IdeS, TCPPCPAPELLG.

11. Composition d'anticorps de la revendication 1 ou la revendication 2 pour utilisation en thérapie.


### SEQUENCE LISTING

<110> Centocor, Inc.
<120> IMMUNOGLOBULIN CLEAVAGE FRAGMENTS AS DISEASE INDICATORS AND COMPOSITIONS
   FOR DETECTING AND BINDING SUCH
<130> CEN5192PCT
<140> TO BE ASSIGNED
   <141> 2008-08-04
<150> 60/955162
   <151> 2007-08-10
<160> 11
<170> PatentIn version 3.5
<210> 1
   <211> 25
   <212> PRT
   <213> Homo sapiens
<400> 1
   Ser Cys Asp Lys Thr His Thr Cys Pro Pro Cys Pro Ala Pro Glu Leu
   1               5                   10                  15
   Leu Gly Gly Pro Ser Val Phe Leu Phe
               20                  25
<210> 2
   <211> 41
   <212> PRT
   <213> Homo Sapiens
<400> 2
   Thr Cys Asn Val Asp His Lys Pro Ser Asn Thr Lys Val Asp Lys Arg
   1               5                   10                  15
   Val Glu Ser Lys Tyr Gly Pro Pro Cys Pro Ser Cys Pro Ala Pro Glu
               20                  25                  30
   Phe Leu Gly Gly Pro Ser Val Phe Leu
           35                  40
<210> 3
   <211> 41
   <212> PRT
   <213> Homo sapiens
<400> 3
   Thr Cys Asn Val Asp His Lys Pro Ser Asn Thr Lys Val Asp Lys Thr
   1               5                   10                  15
   Val Glu Arg Lys Cys Cys Val Glu Cys Pro Pro Cys Pro Ala Pro Pro
               20                  25                  30
   Val Ala Gly Pro Ser Val Phe Leu Phe
           35                  40
<210> 4
   <211> 40
   <212> PRT
   <213> Homo sapiens
<400> 4
   Ser Cys Asp Thr Pro Pro Pro Cys Pro Arg Cys Pro Glu Pro Lys Ser
   1               5                   10                  15
   Cys Asp Thr Pro Pro Pro Cys Pro Arg Cys Pro Ala Pro Glu Leu Leu
               20                  25                  30
   Gly Gly Pro Ser Val Phe Leu Phe
           35                  40
<210> 5
   <211> 14
   <212> PRT
   <213> Homo sapiens
<400> 5
   Ser Cys Asp Lys Thr His Thr Cys Pro Pro Cys Pro Ala Pro
   1               5                   10
<210> 6
   <211> 14
   <212> PRT
   <213> Homo sapiens
<400> 6
   Cys Asp Lys Thr His Thr Cys Pro Pro Cys Pro Ala Pro Glu
   1               5                   10
<210> 7
   <211> 14
   <212> PRT
   <213> Homo sapiens
<400> 7
   Thr His Thr Cys Pro Pro Cys Pro Ala Pro Glu Leu Leu Gly
   1               5                   10
<210> 8
   <211> 14
   <212> PRT
   <213> Homo sapiens
<400> 8
   Thr Lys Val Asp Lys Lys Val Glu Pro Lys Ser Cys Asp Lys
   1               5                   10
<210> 9
   <211> 14
   <212> PRT
   <213> Homo sapiens
<400> 9
   Lys Val Asp Lys Lys Val Glu Pro Lys Ser Cys Asp Lys Thr
   1               5                   10
<210> 10
   <211> 14
   <212> PRT
   <213> Homo sapiens
<400> 10
   Asp Lys Thr His Thr Cys Pro Pro Cys Pro Ala Pro Glu Leu
   1               5                   10
<210> 11
   <211> 14
   <212> PRT
   <213> Homo sapiens
<400> 11
   Val Asp Lys Lys Val Glu Pro Lys Ser Cys Asp Lys Thr His
   1               5                   10